(19) Europäisches Patentamt
European Patent Office
Office européen des brevets

(11) Veröffentlichungsnummer: **0 552 462 A1**

# (12) EUROPÄISCHE PATENTANMELDUNG

(21) Anmeldenummer: **92121171.0**

(22) Anmeldetag: **11.12.92**

(51) Int. Cl.5: **C07D 513/04**, A61K 31/54

(30) Priorität: **13.12.91 DE 4141218**

(43) Veröffentlichungstag der Anmeldung: **28.07.93 Patentblatt 93/30**

(84) Benannte Vertragsstaaten: **AT BE CH DE DK ES FR GB GR IE IT LI LU NL PT SE**

(71) Anmelder: **LUITPOLD PHARMA GmbH**
**Zielstattstrasse 9**
**W-8000 München 70(DE)**

(72) Erfinder: **Strasser, Rupert, Dipl.-Chem.Dr.rer.nat.**
**Römerstrasse 19**
**W-8021 Strasslach(DE)**
Erfinder: **Zeiller, Peter, Dr.med.Vet.**
**Bellinzonastrasse 8**
**W-8000 München 71(DE)**
Erfinder: **Klauser, Rainer, J., Dipl.-CHem,.Dr.rer.nat.**
**Paosostrasse 24**
**W-8000 München 60(DE)**

(74) Vertreter: **Zumstein, Fritz, Dr.**
**Dr. F. Zumstein Dipl.-Ing. F. Klingseisen**
**Bräuhausstrasse 4**
**W-8000 München 2 (DE)**

## (54) Thiadiazincarbonsäureamidderivate, Verfahren zu ihrer Herstellung und Arzneimittel.

(57) Die Erfindung betrifft Thiadiazincarbonsäureamidderivate der allgemeinen Formel **I**,

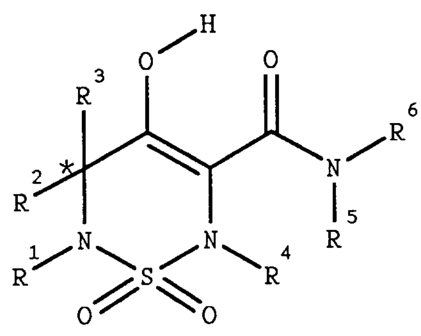

(I)

Verfahren zu deren Herstellung und sie enthaltende Arzneimittel.

EP 0 552 462 A1

Rank Xerox (UK) Business Services
(3.10/3.6/3.3.1)

Die Erfindung betrifft Verbindungen der allgemeinen Formel **I**

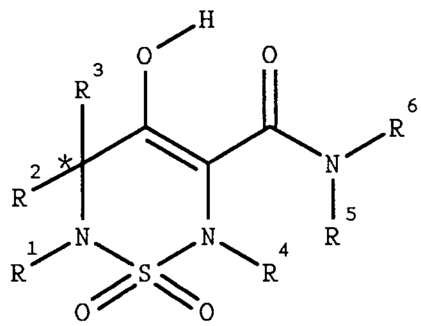

(I)

worin

**$R^1$** einen Niedrigalkylrest, einen Arylrest, einen Heteroarylrest oder einen Arylniedrigalkylrest bedeutet,

**$R^2$** ein Wasserstoffatom, einen Niedrigalkylrest oder einen Arylrest bedeutet,

**$R^3$** ein Wasserstoffatom, einen Niedrigalkylrest, einen Arylrest, einen Heteroarylrest, einen Arylniedrigalkylrest oder einen gesättigten, unverzweigten $C_1$-$C_4$-Alkylrest bedeutet, der mit einem Rest aus der Gruppe **$-OR^7$**, **$-NR^8R^9$**, **$-CO-OR^{10}$**, **$-SR^{11}$**, **$-CO-NR^{12}R^{13}$** oder **$-NH-C(NH_2)(=NH)$** einfach substituiert ist,

oder

worin **$R^1$** und **$R^3$** zusammen einen unverzweigten, gesättigten Alkylenrest mit zwei, drei, vier oder fünf Kohlenstoffatomen bilden und so mit dem benachbarten Stickstoffatom und Kohlenstoffatom des Thiadiazinringsystems einen Ring mit vier, fünf, sechs oder sieben Ringgliedern bilden,

**$R^4$** ein Wasserstoffatom, einen Niedrigalkylrest oder einen Arylniedrigalkylrest bedeutet,

**$R^5$** ein Wasserstoffatom oder einen Niedrigalkylrest bedeutet,

**$R^6$** ein Wasserstoffatom, einen Niedrigalkylrest, einen Arylrest, einen Heteroarylrest oder einen Arylniedrigalkylrest bedeutet,

wobei **$R^7$, $R^8$, $R^9$, $R^{10}$ $R^{11}$, $R^{12}$** und **$R^{13}$** unabhängig voneinander ein Wasserstoffatom, einen Niedrigalkylrest, einen Arylrest oder einen Arylniedrigalkylrest bedeuten,

und deren Salze mit physiologisch verträglichen Säuren und Basen,

wobei der vorstehend genannte Ausdruck Arylrest einen Phenylrest, einen 1-Naphtylrest oder einen 2-Naphtylrest bedeutet, der gegebenenfalls mit ein, zwei oder drei gleichen oder verschiedenen Substituenten aus der Gruppe der Halogenatome, Hydroxylreste, Niedrigalkylreste, Niedrigalkyloxyreste, Carbonsäurereste, Niedrigalkyloxycarbonylreste, Nitrogruppen, Sulfonsäurereste, Trifluormethylreste oder mit Wasserstoffatomen oder Niedrigalkylresten substituierten Aminogruppen substituiert ist,

und wobei

der vorstehend genannte Ausdruck Heteroarylrest einen der folgenden Reste bedeutet: Furanylreste, Thienylreste, Pyrrolylreste, Pyrazolylreste, Imidazolylreste, Triazolylreste, Thiazolylreste, Oxazolylreste, Isothiazolylreste, Isoxazolylreste, Thiadiazolylreste, Pyridylreste, Pyrimidylreste, Pyrazinylreste, Triazinylreste, Benzofuranylreste, Benzothienylreste, Indolylreste, Benzoxazolylreste, Benzothiazolylreste, Benzimidazolylreste, Chinolinylreste oder Isochinolinylreste, wobei vorstehend genannte Reste über jedes Ringkohlenstoffatom mit dem Grundgerüst der Verbindungen der allgemeinen Formel I verknüpft sein können und wobei die vorstehend genannten Ringe gegebenenfalls mit ein oder zwei gleichen oder verschiedenen Substituenten aus der Gruppe der Halogenatome, Hydroxylreste, Niedrigalkylreste, Niedrigalkyloxyreste, Carbonsäurereste, Niedrigalkyloxycarbonylreste, Nitrogruppen, Sulfonsäurereste, Trifluormethylreste oder mit Wasserstoffatomen oder Niedrigalkylresten substituierten Aminogruppen substituiert sein können,

und wobei

der vorstehend genannte Ausdruck Arylniedrigalkylrest einen Methylrest oder einen Ethylrest bedeutet, der mit einem Arylrest substituiert ist, der wie obenstehend definiert ist,

und wobei

der vorstehend genannte Ausdruck Niedrigalkylrest bzw. der Ausdruck "Niedrigalkyl" im Zusammenhang mit Niedrigalkyloxyrest oder Niedrigalkyloxycarbonylrest einen unverzweigten oder verzweigten gesättigten Kohlenwasserstoffrest mit bis zu sechs Kohlenstoffatomen bedeutet.

Die erfindungsgemäßen Verbindungen der allgemeinen Formel **I** liegen im allgemeinen im Sinne einer Keto-Enol-Tautomerie einzeln oder im Gemisch vor.

Für die im Zusammenhang mit der vorliegenden Anmeldungsbeschreibung genannten veschiedenen Substituenten bzw. Reste in Formel **I** gelten folgende Erläuterungen:

Beispiele für **Niedrigalkylreste** sind Methylreste, Ethylreste, n-Propylreste, iso-Propylreste, n-Butylreste, iso-Butylreste, 1-Methylpropylreste, tert.-Butylreste, n-Pentylreste, 1-Methylbutylreste, 2-Methylbutylreste, 3-Methylbutylreste, 1,1-Dimethylpropylreste, 2,2-Dimethylpropylreste, 1,2-Dimethylpropylreste, 1-Ethylpropylreste, n-Hexylreste, 1-Methylpentylreste, 2-Methylpentylreste, 3-Methylpentylreste, 4-Methylpentylreste, 1,1-Dimethylbutylreste, 2,2-Dimethylbutylreste, 3,3-Dimethylbutylreste, 1,2-Dimethylbutylreste, 2,3-Dimethylbutylreste, 1,3-Dimethylbutylreste, 1-Ethylbutylreste, 2-Ethylbutylreste, 1,1,2-Trimethylpropylreste, 1,2,2-Trimethylpropylreste, 1-Ethyl-2-methylpropylreste oder 1-Ethyl-1-methylpropylreste. Dabei sind Methylreste, Ethylreste, n-Propylreste, iso-Propylreste, n-Butylreste, iso-Butylreste, tert.-Butylreste, 2,2-Dimethylpropylreste oder 1-Methylpropylreste bevorzugt. Besonders bevorzugt sind Methylreste, Ethylreste, iso-Propylreste und n-Butylreste.

Beispiele für Arylreste sind Phenylreste, 1-Naphtylreste und 2-Naphtylreste, bei denen gegebenenfalls ein, zwei oder drei gleiche oder verschiedene Substituenten an allen möglichen Positionen des aromatischen Ringsystems vorliegen können. Bevorzugte Arylreste sind Phenylreste, bei denen gegebenenfalls ein, zwei oder drei gleiche oder verschiedene Substituenten an allen möglichen Positionen des aromatischen Ringsystems vorliegen können. Beispiele für **Substituenten** sind Halogenatome, Hydroxylreste, Niedrigalkyloxyreste, Carbonsäurereste, Niedrigalkyloxycarbonylreste, Niedrigalkylreste, Nitrogruppen, Sulfonsäurereste, Trifluormethylreste oder mit Wasserstoffatomen oder Niedrigalkylresten substituierte Aminogruppen. Bevorzugte Substituenten sind Halogenatome, Niedrigalkylreste, Niedrigalkyloxyreste, Niedrigalkyloxycarbonylreste und Trifluormethylreste. Besonders bevorzugte Arylreste sind Phenylreste, 2-Methylphenylreste, 4-Methylphenylreste, 2-Methoxyphenylreste, 4-Methoxyphenylreste, 2-Methoxy-5-methylreste, 2,3-Dimethoxyphenylreste, 2,4-Dimethoxyphenylreste, 2,5-Dimethoxyphenylreste, 2,6-Dimethoxyphenylreste, 3,4-Dimethoxyphenylreste, 3,5-Dimethoxyphenylreste, 2-Ethoxyphenylreste, 4-Ethoxyphenylreste, 2-Chlorphenylreste, 4-Chlorphenylreste, 2,4-Dichlorphenylreste, 2,6-Dichlorphenylreste, 2-Fluorphenylreste, 4-Fluorphenylreste oder 3-Trifluormethylphenylreste. Ganz besonders bevozugte Arylreste sind Phenylreste, 2-Methoxyphenylreste, 4-Methoxyphenylreste, 2-Methoxy-5-methylreste, 2,4-Dimethoxyphenylreste, 2,5-Dimethoxyphenylreste, 4-Ethoxyphenylreste, 4-Chlorphenylreste, 4-Fluorphenylreste oder 3-Trifluormethylphenylreste.

Arylniedrigalkylreste sind Methylreste oder Ethylreste, die mit einem Arylrest substituiert sind, der wie vorstehend definiert ist. Bevorzugte Arylniedrigalkylreste sind Benzylreste und Phenylethylreste, die am Phenylring unsubstituiert oder einfach oder zweifach mit Halogenatomen, Niedrigalkylresten, Niedrigalkyloxyresten, Trifluormethylresten oder Niedrigalkyloxycarbonylresten substituiert sind. Besonders bevorzugte Arylniedrigalkylreste sind Benzylreste, Phenylethylreste, 2-Methylbenzylreste, 4-Methylbenzylreste, 2-Methoxybenzylreste, 4-Methoxybenzylreste, 2-Ethoxybenzylreste, 4-Ethoxybenzylreste, 2-Fluorbenzylreste, 4-Fluorbenzylreste, 2-Chlorbenzylreste, 4-Chlorbenzylreste, 2,4-Dimethoxybenzylreste, 2,5-Dimethoxybenzylreste, 2,4-Dichlorbenzylreste, 2,6-Dichlorbenzylreste, 2-Trifluormethylbenzylreste, 3-Trifluormethylbenzylreste, 4-Trifluormethylbenzylreste, 4-Methoxycarbonylbenzylreste oder 4-Ethoxycarbonylbenzylreste. Ganz besonders bevorzugte Arylniedrigalkylreste sind Benzylreste, Phenylethylreste, 2-Methoxybenzylreste, 4-Methoxybenzylreste, 2-Ethoxybenzylreste, 4-Ethoxybenzylreste, 4-Fluorbenzylreste oder 4-Chlorbenzylreste.

Heteroarylreste sind fünfgliedrige oder sechsgliedrige aromatische Heterocyclen mit einem, zwei oder drei gleichen oder unterschiedlichen Heteroatomen wie Stickstoffatomen, Sauerstoffatomen oder Schwefelatomen, wobei gegebenenfalls ein Benzolring an beliebiger erlaubter Stelle annelliert ist und wobei die Heteroarylreste unsubstituiert oder mit einem oder zwei gleichen oder verschiedenen Substituenten wie Halogenatomen, Hydroxylresten, Niedrigalkyloxyresten, Carbonsäureresten, Niedrigalkyloxycarbonylresten, Niedrigalkylresten, Nitrogruppen, Sulfonsäureresten, Trifluormethylresten oder mit Wasserstoffatomen oder Niedrigalkylresten substituierten Aminogruppen substituiert sein können und wobei die Heteroarylreste über alle Ringkohlenstoffatome mit dem Grundgerüst der Verbindungen der allgemeinen Formel **I** verknüpft sein können. Bevorzugte Heteroarylreste sind die nachstehend genannten Reste, die unsubstituiert oder einfach mit Halogenatomen, Niedrigalkylresten, Niedrigalkyloxyresten oder Nitrogruppen substituiert sein können: Furanylreste, Thienylreste, Pyrrolylreste, Pyrazolylreste, Imidazolylreste, Triazolylreste, Thiazolylreste, Oxazolylreste, Isothiazolylreste, Isoxazolylreste, Thiadiazolylreste, Pyridylreste, Pyrimidylreste, Pyrazinylreste, Triazinylreste, Benzofuranylreste, Benzothienylreste, Indolylreste, Benzoxazolylreste, Benzothiazolylreste, Benzimidazolylreste, Chinolinylreste oder Isochinolinylreste, wobei vorstehend genannte Reste über jedes Ringkohlenstoffatom mit dem Grundgerüst der Verbindungen der allgemeinen Formel I verknüpft sein können. Besonders bevorzugt sind die folgenden Heteroarylreste: 2-Furanylreste, 2-Thienylreste, 3-Thienylreste, 2-Imidazolylreste, 2-Thiazolylreste, 2-Pyridylreste, 4-Pyridylreste, 2-Pyrimidylreste, 3-Isoxazolylreste, 5-Chlor-2-pyridylreste, 5-Methyl-2-pyridylreste, 5-Nitro-2-pyridylreste, 5-Methyl-3-isoxazolylreste, 5-Methyl-2-thiazolylreste und 6-Methoxy-2-benzothiazolylreste. Ganz besonders bevorzugte Heteroarylreste sind 2-Pyridylreste, 2-Thiazolylreste, 5-Methyl-2-thiazolylreste oder 5-Methyl-3-isoxazolylreste.

Beispiele für **Halogenatome** sind Fluor-, Chlor-, Brom- und Iodatome.

Sofern die erfindungsgemäßen Verbindungen in Salzform vorliegen, handelt es sich um die Salze physiologisch verträglicher anorganischer oder organischer Basen und Säuren.

Beispiele für Salze mit physiologisch verträglichen Basen sind Ammonium-, Natrium-, Kalium-, Lithium-, Magnesium- und Calciumsalze, sowie Salze mit Ethanolamin, Triethanolamin, Morpholin oder Piperidin.

Beispiele für Salze mit physiologisch verträglichen Säuren sind Citrat-, Tartrat-, Acetat-, Fumarat-, Gluconat-, Glutamat-, Lactat-, Malat-, Maleat-, Mesylat-, Succinat-, Carbonat-, Bicarbonat-, Bisulfat-, Phosphat-, Hydrogenphosphat-, Diphosphat-, Chlorid- und Bromid-haltige Salze.

Die erfindungsgemäßen Verbindungen haben, außer wenn $\mathbf{R}^2$ und $\mathbf{R}^3$ identische Reste bedeuten, das mit * markierte Kohlenstoffatom als optisch aktives Zentrum und können als optisch reine Enantiomere in der D-Form oder in der L-Form oder als Racemat vorliegen.

Die Synthese von Verbindungen der allgemeinen Formel **I** erfolgt in Analogie zu literaturbekannten Methoden.

Gegenstand der Erfindung ist daher auch ein Verfahren zur Herstellung von Verbindungen der allgemeinen Formel **I**, das dadurch gekennzeichnet ist, daß man in an sich bekannter Weise eine Verbindung der allgemeinen Formel **II**,

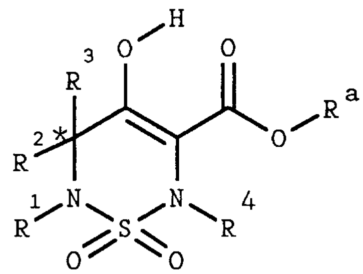

(II)

worin $\mathbf{R}^1$, $\mathbf{R}^2$, $\mathbf{R}^3$ und $\mathbf{R}^4$ die Bedeutungen gemäß Anspruch **1** haben und $\mathbf{R}^a$ einen Methyl- oder Ethylrest bedeutet, umsetzt mit einem Amin der allgemeinen Formel **III**,

$R^5R^6NH$ (III)

worin $\mathbf{R}^5$ und $\mathbf{R}^6$ die Bedeutungen gemäß Anspruch **1** haben.

Nur als Beispiele sind die nachstehenden bekannten Syntheseverfahren zu nennen.

**Stufe 1:**

Nach gängigen Methoden (z.B. K.-H. Deimer, P. Thamm, P. Stelzel in Houben-Weyl, Methoden der Organischen Chemie, Band 15, Teil 1, S. 315 ff, Georg Thieme Verlag, Stuttgart 1974) werden aus α-Aminosäuren α-Aminosäureester, bevorzugt Methyl- und Ethylester, und deren Salze hergestellt, so zum Beispiel nach:

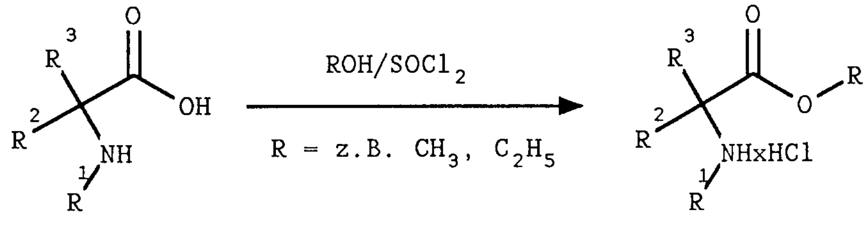


Die Darstellung von am Stickstoffatom mit $\mathbf{R}^1$ substituierten α-Aminosäuren ist ebenso hinreichend bekannt wie die Synthese der verschiedenen, am α-Kohlenstoffatom mit $\mathbf{R}^2$ und/oder $\mathbf{R}^3$ substituierten unnatürlichen Aminosäuren (z.B. Th. Wieland in Houben-Weyl, Methoden der Organischen Chemie, Band XI, Teil 2, S. 305 ff, Georg Thieme Verlag, Stuttgart 1958).

**Stufe 2:**

Die Umsetzung von Chlorsulfonylisocyanat mit Benzylalkohol und anschließende Reaktion mit α-Aminosäureestern oder mit α-Aminosäureester-Hydrochloriden unter Verwendung tertiärer Aminobasen liefert N-[N'-(Benzyloxycarbonyl)sulfamoyl]-α-aminosäureester.

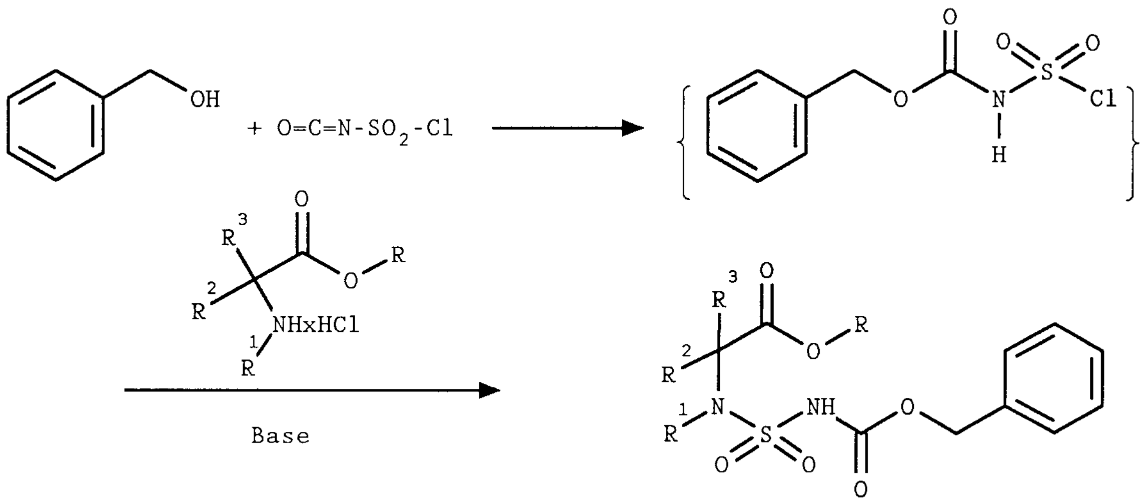


**Stufe 3:**

Durch anschließende katalytische Hydrierung erhält man die entsprechend unsubstituierten N-Sulfamoyl-α-aminosäureester.

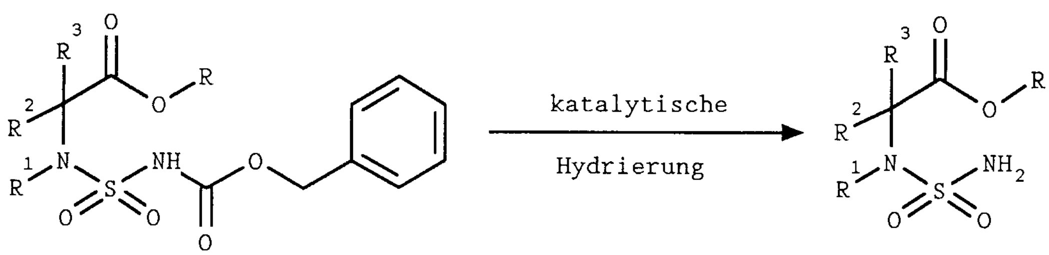


**Stufe 4:**

Mit Natriummethanolat erfolgt beim Erhitzen unter ROH-Abspaltung der Ringschluß zum entsprechenden 1,1-Dioxo-1,2,5-thiadiazolidin-3-on.

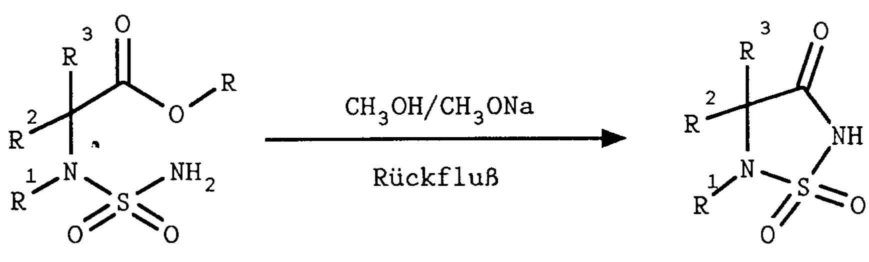


Beispiele für Reaktionen gemäß Stufen 2 bis 4 sind in der Literatur bei G. W. Muller, G. E. DuBois, J. Org. Chem. **54**, 4471 (1989) beschrieben.

Weitere Zugangsmöglichkeiten zu 1,1-Dioxo-1,2,5-thiadiazolidin-3-on-Derivaten (Derivaten der Stufe 4) sind beispielsweise noch bei H. K. Vorreither, E. Ziegler, Monatsh. Chem. **96**, 216 (1965), bei J. W. Timberlake, W. J. Ray Jr., E. D. Stevens, J. Org. Chem. **54**, 5824 (1989), sowie bei C.-H. Lee, J. D. Korp, H. Kohn, J. Org. Chem. **54**, 3077 (1989) beschrieben.

Einen Zugangsweg zu annellierten Verbindungen der Stufe 4 aus cyclischen Aminosäurederivaten beschreiben C.-H. Lee und H. Kohn in J. Org. Chem. **55**, 6098 (1990).

**Stufe 5:**

Die Alkylierung mit Halogenessigsäureestern, bevorzugt mit Bromessigsäuremethylester, führt zu den 2-N-alkylierten Fünfringsystemen.

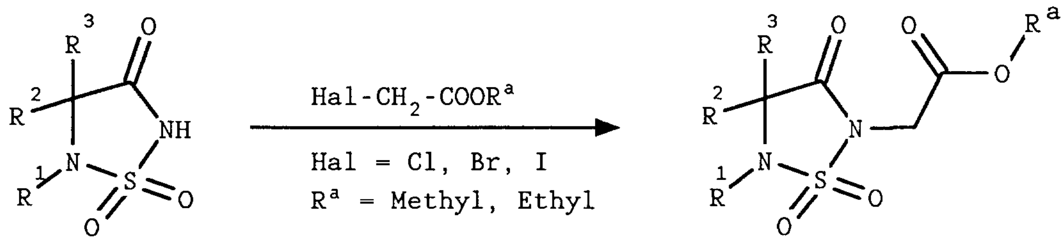


**Stufe 6:**

Durch basische Umlagerung des Essigsäurederivates gelangt man zum Dihydrothiadiazingerüst.

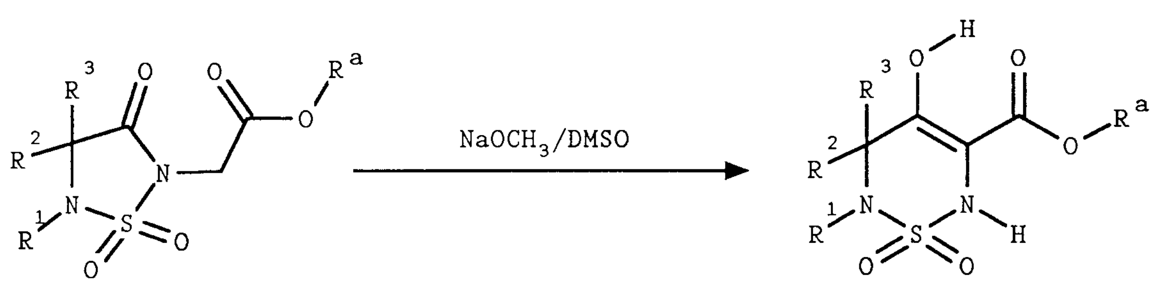


**Stufe 7, (fakultativ):**

Bei der Herstellung von Verbindungen der allgemeinen Formel **I**, bei denen $\mathbf{R^4}$ eine andere Bedeutung als ein Wasserstoffatom besitzt, erfolgt die Umsetzung mit Verbindungen der allgemeinen Formel $\mathbf{R^bHal}$, worin $\mathbf{R^b}$ die Bedeutung Niedrigalkyl oder Arylniedrigalkyl besitzt, zu den entsprechenden N-substituierten Alkylderivaten.

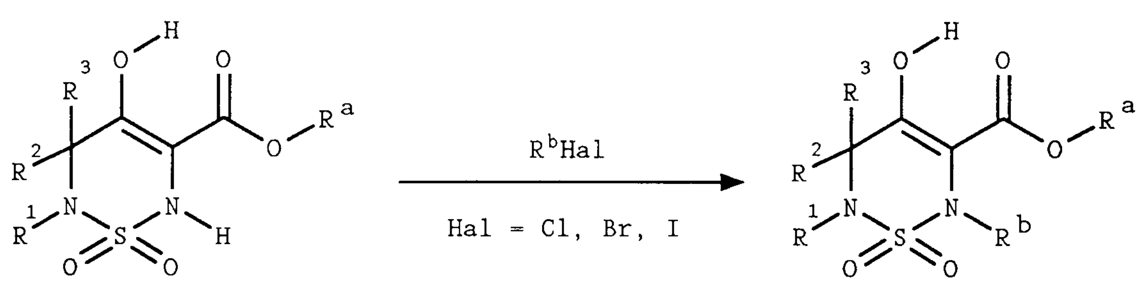

**Stufe 8:**

Unter Abspaltung von $R^aOH$ erhält man aus Verbindungen der Stufen 6 oder 7 durch Umsetzung mit Aminen in hochsiedenden aromatischen Kohlenwasserstoffen, gegebenenfalls unter Verwendung allgemein üblicher Amidierungskatalysatoren, die erfindungsgemäßen Verbindungen der allgemeinen Formel **I**.

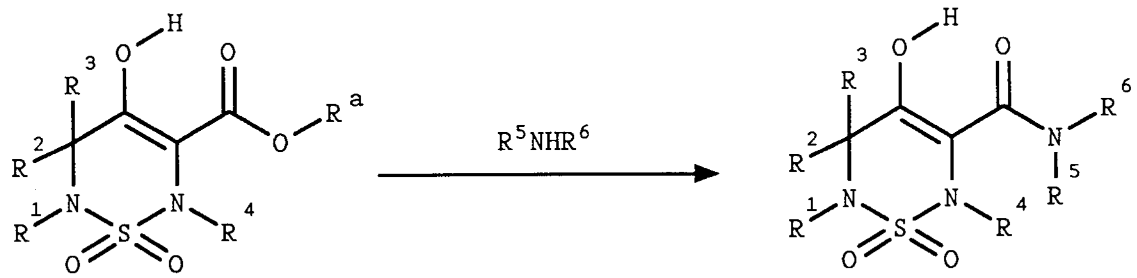


Die Stufen 5 bis 8 werden in Anlehnung an publizierte Synthesen durchgeführt (J. G. Lombardino, E. H. Wiseman, W. M. McLamore, J. Med. Chem. **14**, 1171 (1971), US-Patent 3 591 584, DOS 1 943 265).

Alternativ können die erfindungsgemäßen Verbindungen ab Stufe 5 gemäß folgender Verfahrensvariante erhalten werden:

**Stufe 5, Variante:**

Verwendet man in obiger Stufe 5 statt Halogenessigsäureester Halogenessigsäureamide, die leicht aus α-Halogencarbonsäurehalogeniden und Aminen erhältlich sind, gelangt man nach Umsetzung mit 1,1-Dioxo-1,2,5-thiadiazolidin-3-onen zu den entsprechenden 2-(2-Acetylamido)-1,1-dioxo-1,2,5-thiadiazolidin-3-onen.

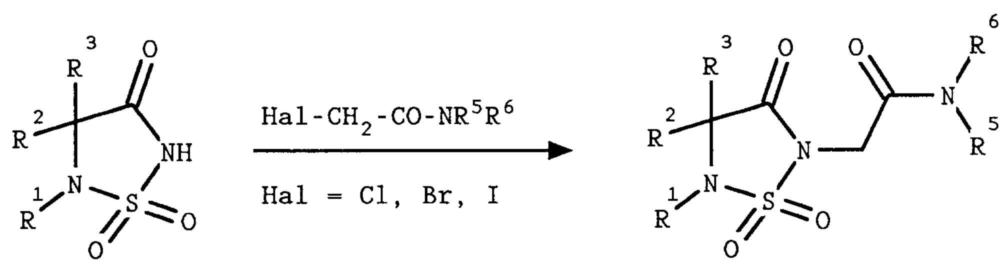


**Stufe 6, Variante:**

Auch hier erfolgt die basische Umlagerung vom Fünfring zum Sechsring wie in obiger Stufe 6.

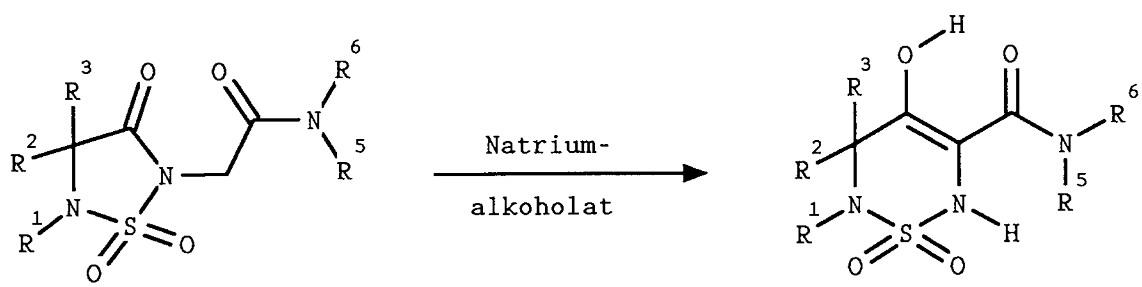

**Stufe 7, Variante (fakultativ):**

Bei der Herstellung von Verbindungen der allgemeinen Formel **I**, bei denen $\mathbf{R}^4$ eine andere Bedeutung als ein Wasserstoffatom besitzt, erfolgt die Umsetzung mit Verbindungen der allgemeinen Formel $\mathbf{R}^b\mathbf{Hal}$, worin $\mathbf{R}^b$ die Bedeutung Niedrigalkyl oder Arylniedrigalkyl besitzt, zu den entsprechenden N-substituierten Alkylderivaten.

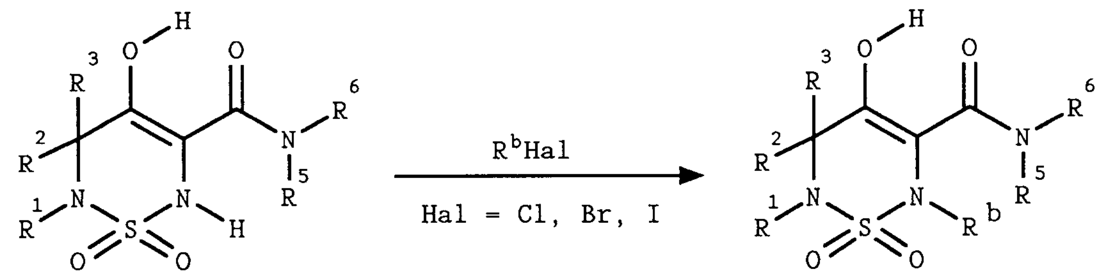


Diese Reaktionssequenz ist ebenfalls (I. A. Perillo, C. B. Schapira, S. Lamdan, J. Heterocyclic Chem. **20**, 155 (1983) beschrieben.

In beiden Varianten werden unabhängig davon, ob in Stufe 1 enantiomerenreine oder racemische Aminosäuren eingesetzt werden, im allgemeinen racemische Gemische der Zielverbindungen erhalten. Diese können nach üblichen Verfahren (z.B. Wilen, Top. Stereochem. **6**, 107 (1971), Wilen, Collet, Jacques, Tetrahedron **33**, 2725 (1977), Boyle, Q. Rev. Chem. Soc. **25**, 323 (1971)), beispielsweise durch Überführung in diastereomere Enolether, Enolester, Acetale oder Ketale mit optisch reinen Alkoholen, Säuren, Aldehyden, Ketonen oder deren reaktiven Derivaten, anschließende Trennung der so gebildeten Diastereomeren beispielsweise durch fraktionierte Kristallisation oder durch chromatographische Methoden, zuletzt durch Abspaltung der chiralen Hilfsgruppen, oder durch übliche chromatographische Methoden unter Verwendung chiraler Säulenmaterialien, in die enantiomerenreinen Verbindungen überführt werden.

Sofern die in Stufe 1 verwendeten Aminosäuren bei $\mathbf{R}^3$ funktionelle Gruppen aufweisen, müssen diese gegebenenfalls nach allgemein üblichen Methoden (z.B. Houben-Weyl, Methoden der Organischen Chemie, Band 15, Georg Thieme Verlag, Stuttgart 1974; Th. W. Greene, Protective Groups in Organic Synthesis, John Wiley & Sons, New York 1981) geschützt werden. Gleiches gilt für den Rest $\mathbf{R}^6$, wenn er eine funktionelle Gruppe enthält, und wenn derartige Verbindungen der allgemeinene Formel **I** nach der obigen Verfahrensvariante hergestellt werden.

Die erfindungsgemäßen Verbindungen der allgemeinen Formel **I** stellen neue Substanzen dar.

Aus J. Chem. Res.(S), **1988**, 94 - 95, ferner aus Chem. Abstr. **104**, 207234 (1986) sowie aus Chem. Abstr. **107**, 89296 (1987) und Chem. Abstr. **106**, 5093 (1987) sind Thiadiazinone bekannt. Aus J. Org. Chem. **29**, 1905 - 1909 (1964), ferner aus US-Patent 2 956 997, US-Patent 3 223 703 und US-Patent 3 203 954 sind gewisse Thiadiazine bekannt. Aus J. Med. Chem. **14**, 1171 - 1175 (1971) und aus J. Med. Chem. **16**, 493 - 496 (1973) sind gewisse Benzothiazine , die sogenannten Oxicame bekannt. Für vereinzelte Vertreter aus den Klassen der Thiadiazinone und der Thiadiazine sind pharmakologische Wirkungen beschrieben. Die Klasse der Oxicame umfaßt potente antientzündliche Substanzen, beispielsweise das Piroxicam, die jedoch mit einigen Nachteilen behaftet sind (Scrip **19**,984 (1985) oder Drugs **35**, 504 (1988)). Strukturell den erfindungsgemäßen Verbindungen nahestehende Substanzen sind nicht bekannt geworden.

Die Verbindungen der allgemeinen Formel **I** stellen wertvolle pharmazeutische Wirkstoffe dar.

Überraschenderweise zeichnen sich die erfindungsgemäßen Verbindungen durch eine starke antientzündliche Wirkung aus und sind frei von unerwünschten Eigenschaften. Außerdem haben diese Verbindungen starke analgetische und antipyretische Eigenschaften. Besonders hervorzuheben ist die gute Verträglichkeit und das Fehlen ulcerogener Eigenschaften. Deshalb eignen sich die erfindungsgemäßen Verbindungen zur Behandlung entzündlicher und schmerzhafter Erkrankungen bei Mensch und Tier. Die Krankheitsbilder, die mit den erfindungsgemäßen Verbindungen behandelt werden können, umfassen alle entzündlichen Zustände, wie zum Beispiel Entzündungen der Haut wie Radiodermatitis, Psoriasis, Akne, Ekzeme, Entzündungen am Auge wie zum Beispiel Conjunktivitis, Entzündungen im Hals-Nasen-Ohren-Bereich wie zum Beispiel katarralische Infektionen der oberen Luftwege und Sinusitis, entzündliche Erkrankungen des Gefäßsystems wie Thrombophlebitis oder Vaskulitis und Entzündungen des Nervensystems wie zum Beispiel Neuralgien oder Neuritiden. Bevorzugt zu behandelnde entzündliche Erkrankungen sind Entzündungen im Bereich des Bewegungsapparates wie Arthritiden, Arthrose, Tendinitis, Tendopathien, Kontusionen und Distorsionen, Weichteilrheumatismus, Gicht und Lumbalgien. Die erfindungsgemäßen Verbindungen eignen sich auch besonders zur Behandlung von bestimmten Erkrankungen oder Störungen, bei denen der Schmerz im Vordergrung steht. Hier sind postoperative Schmerzzustände, Schmerzen nach Zahnextraktion und Migräne zu nennen.

In Untersuchungen auf antientzündliche Wirkung (gemäß C. A. Winter, E. A. Risley, G. W. Nuss; Proc. Soc. Exp. Biol. Med. **111**, 544 (1962)) an Gruppen mit je zehn Wistar-Ratten erwiesen sich erfindungsgemäße Verbindungen als stark antientzündlich wirksam. Beispielsweise zeigt die Verbindung des nachstehenden Beispiels 4 bei einer Dosierung von 200 mg/kg Körpergewicht p.o. eine Hemmung von 42 %.

Auch bei der Prüfung auf analgetische Eigenschaften (gemäß R. A. Turner, Screening Methods in Pharmacology, Academic Press, New York and London 1965, S. 113) erwiesen sich erfindungsgemäße Verbindungen als stark wirksam. Beispielsweise zeigte die Verbindung aus Beispiel 4 einen 100 %igen Schutzeffekt bei einer Dosierung von 200 mg/kg Körpergewicht p.o.

Die erfindungsgemäßen Verbindungen können in einer Vielzahl pharmazeutischer Zubereitungsformen appliziert werden, wie zum Beispiel Tabletten, Dragees, Kapseln, flüssige oral einzunehmende Zubereitungen, Salben, Gele, Pflaster, Injektionslösungen oder Sprays, wobei allgemein übliche Träger- und Hilfsstoffe verwendet werden können, die mit den erfindungsgemäßen Verbindungen verträglich sind.

Gegenstand der vorliegenden Erfindung sind auch pharmazeutische Zubereitungen (Arzneimittel), die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Bevorzugte pharmazeutische Zubereitungen sind Tabletten, Dragees, Kapseln, Granulate, Suppositorien, Lösungen, Suspensionen und Emulsionen, Pasten, Salben, Gele, Cremes, Lotionen, Puder, Sprays und Aerosole.

Tabletten, Dragees, Kapseln und Granulate können den oder die Wirkstoffe neben den üblichen Trägerstoffen enthalten. Dazu gehören a) Füll- und Streckmittel, zum Beispiel Stärken, Milchzucker, Rohrzucker, Glucose, Mannit und Kieselsäure, b) Bindemittel, zum Beispiel Carboxymethylcellulose, Alginate, Gelatine, Polyvinylpyrrolidon, c) Feuchthaltemittel, zum Beispiel Glycerin, d) Sprengmittel, zum Beispiel Agar-Agar, Calciumcarbonat und Natriumbicarbonat, e) Lösungsverzögerer, zum Beispiel Paraffin, f) Resorptionsbeschleuniger, g) Netzmittel, zum Beispiel Cetylalkohol, Glycerinmonostearat, h) Adsorptionsmittel, zum Beispiel Kaolin und Bentonit und i) Gleitmittel, zum Beispiel Talkum, Calcium- und Magnesiumstearat und feste Polyethylenglycole oder Gemische der unter a) bis i) aufgeführten Stoffe.

Die Tabletten, Dragees, Kapseln und Granulate können mit üblichen gegebenenfalls Opalisierungsmittel enthaltenden Überzügen, zum Beispiel Zucker, Überzugslacken, versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen zum Beispiel Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, zum Beispiel Polyethylenglycole, Fette, zum Beispiel Kakaofett und höhere Ester (zum Beispiel $C_{14}$-Alkohol mit $C_{16}$-Fettsäure oder Gemische dieser Stoffe).

Cremes enthalten neben dem oder den Wirkstoffen als ölige Grundlage in erster Linie Fettalkohole, zum Beispiel Lauryl-, Cetyl-, Stearylalkohol, Fettsäuren, zum Beispiel Palmitin- oder Stearinsäure, flüssige bis feste Wachse, zum Beispiel Isopropylmyristat, Wollwachse oder Bienenwachs und/oder Kohlenwasserstoffe, zum Beispiel Vaseline (Petrolatum) oder Paraffinöl. Als Emulgatoren verwendet man bevorzugt solche mit vorwiegend hydrophilen Eigenschaften, zum Beispiel nichtionische Emulgatoren wie Fettsäureester von Polyalkoholen, Ethylenoxidaddukte davon wie Polyglycerinfettsäureester oder Polyoxyethylensorbitanfettsäureester (Tweens) oder ionische Emulgatoren wie Alkalimetallsalze von Fettalkoholsulfaten, zum Beispiel Natriumlaurylsulfat, Natriumcetylsulfat oder Natriumstearylsulfat. Zur Wasserphase können Mittel zugesetzt werden, welche die Austrocknung der Creme verhindern, zum Beispiel Polyalkohole wie Glycerin, Sorbit, Propylenglycol und/oder Polyethylenglycole.

Für Salben kommen neben dem oder den Wirkstoffen als Trägerstoffe in erster Linie Kohlenwasserstoffe, zum Beispiel Vaseline oder Paraffinöl in Frage, die zur Verbesserung des Wasserbindungsvermögens vorzugsweise geeignete Fettalkohole oder Ester davon, zum Beispiel Cetylalkohol oder Wollwachse enthalten. Emulgatoren sind entsprechende lipophile Substanzen wie Sorbitanfettsäureester. Zur Wasserphase können Feuchthaltungsmittel wie zum Beispiel Glycerin oder Propylenglycol zugesetzt werden.

Sprays und Puder können neben dem oder den Wirkstoffen die üblichen Trägerstoffe enthalten, zum Beispiel Milchzucker, Talkum, Kieselsäure, Aluminiumhydroxid, Calciumsilicat und Polyamidpulver oder Gemische dieser Stoffe. Sprays können zusätzlich die üblichen Treibmittel enthalten.

Lösungen und Emulsionen können neben dem oder den Wirkstoffen die üblichen Trägerstoffe wie Lösungsmittel, Lösungsvermittler und Emulgatoren, zum Beispiel Wasser, Ethanol, Isopropanol, Ethylcarbonat, Benzylalkohol, Benzylbenzoat, Propylenglycol, 1,3-Butylenglycol, Öle, insbesondere Baumwollsaatöl, Erdnußöl, Maisöl, Ricinusöl, Cashewnußöl und Sesamöl, Glycerin, Glycerinformal, Polyethylenglycole und Fettsäureester des Sorbitans oder Gemische dieser Stoffe enthalten.

Suspensionen können neben dem oder den Wirkstoffen die üblichen Trägerstoffe wie flüssige Verdünnungsmittel, zum Beispiel Ethanol, Propylenglycol, Suspendiermittel, zum Beispiel ethoxylierte Isostearylalkohole, Polyoxyethylensorbit- und Sorbitanester, mikrokristalline Cellulose, Aluminiummetahydroxid, Bentonit, Agar-Agar oder Traganth oder Gemische dieser Stoffe enthalten.

Die genannten Formulierungsformen können auch Färbemittel, Konservierungsstoffe sowie geruchs- und geschmacksverbessernde Zusätze, zum Beispiel Pfefferminzöl und Eucalyptusöl und Süßmittel, zum Beispiel Saccharin enthalten.

Die therapeutisch wirksamen Verbindungen sollen in den oben aufgeführten pharmazeutischen Zubereitungen vorzugsweise in einer Konzentration von etwa 0.1 bis 99.5, vorzugsweise von etwa 0.5 bis 95 Masse-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, zum Beispiel durch Mischen der Wirkstoffe mit den Trägersoffen.

Die vorliegenden Wirkstoffe bzw. pharmazeutischen Zubereitungen, die einen oder mehrere Wirkstoffe enthalten, können in der Human- und Veterinärmedizin zur Verhütung, Besserung und/oder Heilung entzündlicher Krankheiten eingesetzt werden.

Im allgemeinen erweist es sich als vorteilhaft, in der Humanmedizin den oder die erfindungsgemäßen Wirkstoffe in Gesamtdosen von etwa 1 bis etwa 1000 mg, vorzugsweise 10 bis 500 mg pro Dosiseinheit bei Verabreichung von ein bis vier Dosiseinheiten pro Tag zur Erzielung der gewünschten Ergebnisse zu applizieren.

Es kann jedoch erforderlich sein, von den genannten Dosierungen abzuweichen, und zwar in Abhängigkeit von der Art und dem Körpergewicht des zu behandelnden Objektes, der Art und der Schwere der Erkrankung, der Art der Zubereitung und der Applikation des Arzneimittels, sowie dem Zeitraum bzw. Intervall, innerhalb welchem die Verabreichung erfolgt. So kann es in einigen Fällen ausreichend sein, mit weniger als der oben genannten Wirkstoffmenge auszukommen, während in anderen Fällen die oben angeführte Menge Wirkstoff überschritten werden muß.

Die nachfolgenden Beispiele erläutern die Erfindung näher, ohne ihren Umfang darauf zu beschränken.

Beispiele

**Beispiel 1:**

**5,6-Dihydro-4-hydroxy-2-methyl-1,1-dioxo-N-phenyl-2H-pyrrolidino[1,2-e][1,2,6]thiadiazin-3-carbonsäureamid**

128 mmol 5,6-Dihydro-4-hydroxy-2-methyl-1,1-dioxo-2H-pyrrolidino[1,2-e][1,2,6]thiadiazin-3-carbonsäuremethylester werden mit 1.0 eq frisch destilliertem Anilin in 300 ml trockenem Xylol vorgelegt. Bei 150 bis 160 °C Ölbadtemperatur wird das bei der Reaktion freiwerdende Methanol zusätzlich durch Einleiten eines leichten Stickstoffstromes über eine Destillationsbrücke entfernt. Fehlendes Xylol im Reaktionsansatz wird gegebenenfalls ergänzt. Der nach dem Abkühlen im Ansatz ausgefallene Niederschlag wird abfiltriert, mit Xylol und Diisopropylether nachgewaschen und aus einer Diisopropylether-Aceton-Mischung umkristallisiert. Alkalische Extraktion der Xylollösung, Ansäuern der wäßrigen Lösung und nochmalige Extraktion mit Essigsäureethylester liefert nach Waschen mit Kochsalzlösung, Trocknen und Abziehen des Lösungsmittels weiteres kristallines Rohprodukt, das ebenfalls umkristallisiert wird. Man erhält so in durchschnittlich 50 %iger Ausbeute die gewünschte Titelverbindung.

Schmp.: 163.5 - 164.5 °C

$^1$H-NMR ($CDCl_3$): $\delta$ = 1.75 - 2.64 (m, 4H, 5-$CH_2$ und 6-$CH_2$), 3.12 (s, 3H, $CH_3$), 3.23 - 4.06 (m, 2H, 7-$CH_2$), 4.36 (t, J = 8 Hz, 1H, 4a-CH), 7.03 - 7.69 (m, 5H, Ph-H), 8.10 (s-breit, 1H, NH), 13.05 (s-breit, 1H, OH).

IR (KBr): 3340, 3030 - 2890, 1645, 1595, 1535, 1490, 1445, 1365, 1325, 1300, 1235, 1185, 1150, 1080,

1050, 1030, 1000, 880, 865, 815, 770, 760, 700, 630 $cm^{-1}$.
MS: m/e = 323 ($M^{+\cdot}$), 231, 204, 162, 161, 134, 133, 120, 93 (100 %), 84, 77, 70, 69.

| Elementaranalyse: ($C_{14}H_{17}N_3O_4S$) | | | | |
|---|---|---|---|---|
| | % C | % H | % N | % S |
| berechnet: | 52.00 | 5.30 | 13.00 | 9.91 |
| gefunden: | 51.91 | 5.19 | 12.89 | 10.01 |

**Beispiel 2:**

**5,6-Dihydro-4-hydroxy-N-(2-methoxy-5-methylphenyl)-2-methyl-1,1-dioxo-2H-pyrrolidino[1,2-e][1,2,6]-thiadiazin-3-carbonsäureamid**

Die Darstellung erfolgt analog Beispiel 1 mit 1.0 eq 2-Methoxy-5-methylanilin anstelle von Anilin. Nach Umkristallisation aus Essigsäureethylester wird die Titelverbindung in 69 %iger Ausbeute erhalten.
Schmp.: 163.5 - 164.5 °C
$^1$H-NMR ($CDCl_3$): $\delta$ = 1.66 - 2.63 (m, 4H, 5-$CH_2$ und 6-$CH_2$), 2.36 (s, 3H, Ph-$CH_3$), 3.15 (s, 3H, N-$CH_3$), 3.25 - 4.03 (m, 2H, 7-$CH_2$), 3.91 (s, 3H, O-$CH_3$), 4.33 (t, J = 8 Hz, 1H, 4a-CH), 6.61 - 7.00 (m, 2H, 2 Ar-H), 8.09 (s, 1H, 1 Ar-H), 8.56 (s-breit, 1H, NH).
IR (KBr): 3350, 2940, 1635, 1595, 1540, 1485, 1450, 1440, 1365, 1345, 1315, 1290, 1250, 1225, 1215, 1185, 1170, 1135, 1080, 1025, 1000, 930, 920, 895, 875, 840, 810, 765, 720, 690, 630 $cm^{-1}$.
MS: m/e = 367 ($M^{+\cdot}$), 191, 175, 149, 138, 137 (100 %), 122, 106.

**Beispiel 3:**

**5,6-Dihydro-4-hydroxy-N-(2-methoxyphenyl)-2-methyl-1,1-dioxo-2H-pyrrolidino[1,2-e][1,2,6]thiadiazin-3-carbonsäureamid**

Die Darstellung erfolgt analog Beispiel 1 mit 1.0 eq o-Anisidin anstelle von Anilin. Nach Umkristallisation aus einer Diisopropylether/Aceton-Mischung erhält man die Titelverbindung in 80 %-iger Ausbeute.
Schmp.: 161 - 162.5 °C
$^1$H-NMR ($d_6$-DMSO): $\delta$ = 1.59 - 2.64 (m, 4H, 5-$CH_2$ und 6-$CH_2$), 2.99 - 4.07 (m, 2H, 7-$CH_2$), 3.11 (s, 3H, N-$CH_3$), 3.87 (s, 3H, O-$CH_3$), 4.38 (t, J = 7 Hz, 1H, 4a-CH), 6.81 - 7.37 (m, 3H, 3 Ar-H), 7.64 - 7.97 (m, 1H, 1 Ar-H), 9.13 (s, 1H, NH).
IR (KBr): 3370, 2970, 1635, 1595, 1540, 1485, 1460, 1435, 1365, 1340, 1305, 1290, 1250, 1210, 1175, 1165, 1125, 1070, 1050, 1025, 995, 940, 915, 860, 850, 630 $cm^{-1}$.

**Beispiel 4:**

**5,6-Dihydro-4-hydroxy-2-methyl-1,1-dioxo-N-(2-pyridyl)-2H-pyrrolidino[1,2-e][1,2,6]thiadiazin-3-carbonsäureamid**

Die Darstellung erfolgt analog Beispiel 1 mit 1.0 eq 2-Aminopyridin anstelle von Anilin. Nach Umkristallisation aus Aceton wird die Titelverbindung in 44 %iger Ausbeute erhalten.
Schmp.: 172.5 - 173 °C
$^1$H-NMR ($d_6$-DMSO): $\delta$ = 1.66 - 2.43 (m, 4H, 5-$CH_2$, 6-$CH_2$), 2.76 und 3.06 (2s, 3H, $CH_3$), 3.17 - 3.86 (m, 2H, 7-$CH_2$), 4.35 (t, J = 7 Hz, 1H, 4a-CH), 4.87 (s, 1H, 3-CH), 7.00 - 7.35 (m, 1H, 1Ar-H), 7.66 - 8.12 (m, 2H, 2Ar-H), 8.21 - 8.46 (m, 1H, 1Ar-H), 10.69 (s-breit, 1H, NH), Keto : Enol = 1 : 5.
IR (KBr): 3440, 3160, 2980, 2880, 1640, 1590, 1575, 1535, 1510, 1435, 1365, 1340, 1330, 1305, 1285, 1235, 1210, 1170, 1140. 1065, 1030, 995, 930, 890, 815, 775, 740, 635, 625 $cm^{-1}$.
MS: m/e = 324 ($M^{+\cdot}$), 260, 204, 191, 164, 163, 148, 138, 134, 123, 122, 121, 94 (100 %), 78,70,69,68,67,55.

**Beispiel 5:**

**5,6-Dihydro-4-hydroxy-2-methyl-N-(5-methyl-3-isoxazolyl)-1,1-dioxo-2H-pyrrolidino[1,2-e][1,2,6]-thiadiazin-3-carbonsäureamid**

Die Darstellung erfolgt analog Beispiel 1 mit 1.0 eq 3-Amino-5-methylisoxazol anstelle von Anilin. Nach Umkristallisation aus Essigsäureethylester wird die Titelverbindung in 48 %iger Ausbeute erhalten.
Schmp.: 194 °C; unter Zersetzung
$^1$H-NMR ($d_6$-DMSO): $\delta$ = 1.71 - 2.48 (m, 4H, 5-$CH_2$, 6-$CH_2$), 2.40 (s, 3H, Ar-$CH_3$), 2.74 und 3.00 (2s, 3H, N-$CH_3$), 3.31 - 3.92 (m, 2H, 7-$CH_2$), 4.40 (t, J = 7 Hz, 1H, 4a-CH), 4.69 (s, 1H, 3-CH), 6.56 (s, 1H, Ar-H), 10.84 (s, 1H, NH), Keto : Enol = 1 : 6.
IR (KBr): 3420, 3190, 3060, 2990, 2960, 2930, 2890, 1635, 1610, 1530, 1470, 1440, 1370, 1340, 1320, 1270, 1245, 1210, 1185, 1165, 1150, 1075, 1055, 1030, 1010, 995, 925, 915, 875, 800, 755, 750, 630 $cm^{-1}$.
MS: m/e = 328 ($M^{+\cdot}$), 204, 195, 180, 167, 162, 152, 139, 126, 125, 124, 98, 84, 83, 82, 70 (100 %), 69, 68, 55.

**Beispiel 6:**

**5,6-Dihydro-4-hydroxy-2-methyl-1,1-dioxo-N-(2-thiazolyl)-2H-pyrrolidino[1,2-e][1,2,6]thiadiazin-3-carbonsäureamid**

Die Darstellung erfolgt analog Beispiel 1 mit 1.0 eq 2-Aminothiazol anstelle von Anilin. Nach säulenchromatographischer Aufreinigung über Kieselgel mit einem Fließmittelgemisch aus Diisopropylether/Essigsäureethylester/Ameisensäure = 150/50/1 wird die Titelverbindung in 44 %iger Ausbeute erhalten.
Schmp.: 166 - 168.5 °C
$^1$H-NMR ($d_6$-DMSO): $\delta$ = 1.56 - 2.61 (m, 4H, 5-$CH_2$, 6-$CH_2$), 2.74 und 3.05 (2s, 3H, N-$CH_3$), 3.05 - 3.87 (m, 2H, 7-$CH_2$), 4.33 (t, J = 7 Hz, 1H, 4a-CH), 4.79 (s, 1H, 3-CH), 7.18 (d, J = 4 Hz, 1H, 1 Ar-H), 7.56 (d, J = 4 Hz, 1H, 1 Ar-H), Keto : Enol = 1 : 12.
IR (KBr): 3100, 2970, 1630, 1530, 1485, 1440, 1425, 1365, 1340, 1325, 1270, 1240, 1210, 1190, 1165, 1145,1060, 1030, 990, 945, 930, 915, 895, 825, 785, 760, 720, 680, 670, 625 $cm^{-1}$.

**Beispiel 7:**

**N-Benzyl-5,6-dihydro-4-hydroxy-2-methyl-1,1-dioxo-2H-pyrrolidino[1,2-e][1,2,6]thiadiazin-3-carbonsäureamid**

Die Darstellung erfolgt analog Beispiel 1 mit 1.0 eq Benzylamin anstelle von Anilin. Nach Waschen des kristallinen Rohproduktes mit einem Gemisch aus Diisopropylether/tert.-Butylmethylether wird die Titelverbindung in 11 %iger Ausbeute erhalten.
Schmp.: 123 - 126 °C
$^1$H-NMR ($d_6$-DMSO): $\delta$ = 1.59 - 2.36 (m, 4H, 5-$CH_2$, 6-$CH_2$), 2.89 - 3.92 (m, 2H, 7-$CH_2$), 2.97 (s, 3H, N-$CH_3$), 4.13 - 4.56 (m, 3H, $CH_2$-Ph und 4a-CH), 7.30 (s, 5H, Ph-H), 8.86 (t-breit, J = 7 Hz, 1H, NH).
IR (KBr): 3330, 3030, 2970, 2920, 1635, 1535, 1495, 1450, 1365, 1335, 1235, 1200, 1170, 1145, 1095, 1070, 1025, 995, 985, 915, 865, 825, 765, 740, 700, 690, 665, 620 $cm^{-1}$.

Die in den **Beispielen 1** bis **7** als Ausgangsprodukt verwendete Verbindung 5,6-Dihydro-4-hydroxy-2-methyl-1,1-dioxo-2H-pyrrolidino[1,2-e][1,2,6]thiadiazin-3-carbonsäuremethylester wurde wie in den folgenden Stufen A bis G beschrieben hergestellt:

**Stufe A:**

**Pyrrolidin-2-carbonsäuremethylester-Hydrochlorid**

In einem Mehrhalskolben mit Tropftrichter und Innenthermometer werden 600 ml technisches Methanol unter Eis-Kochsalz-Kühlung vorgelegt. Man läßt 2.4 mol Thionylchlorid so zutropfen, daß die Temperatur der Reaktionslösung -5 °C nicht übersteigt. Danach werden 2.18 mol Prolin portionsweise über etwa 10 Minuten zugegeben. Nach etwa vierstündigem Rühren bei 40 °C und anschließendem dreitägigen Rühren bei Raumtemperatur wird die Lösung weitgehend eingeengt, der Rückstand zur Entfernung überschüssigen

Thionylchlorides mehrmals in Methanol aufgenommen und erneut eingeengt. Nach mehrstündigem Trocknen in einem lauwarmen Wasserbad im evakuierten Kolben erhält man die Zielverbindung in quantitativer Ausbeute. Das ölige Rohprodukt kristallisiert nach mehrtägigem Stehenlassen teilweise aus.
$^1$H-NMR ($d_4$-Methanol): $\delta$ = 1.81 - 2.69 (m, 4H, 3-$CH_2$, 4-$CH_2$), 3.23 - 3.59 (m. 2H, 5-$CH_2$), 3.84 (s, 3H, $CH_3$), 4.46 (t, J = 7 Hz, 1H, 2-CH), 4.81 (s, 2H, NH, HCl).
IR (Ölfilm): 3390 (breit), 2959 (breit), 2740, 1745, 1570, 1445, 1245, 1050, 1000, 920 $cm^{-1}$.

**Stufe B:**

**N-[N-(Benzyloxycarbonyl)sulfamoyl]pyrrolidin-2-carbonsäuremethylester**

In einem Dreihalskolben mit Tropftrichter und Innenthermometer werden unter Schutzgas 275 mmol Chlorsulfonylisocyanat in 300 ml trockenem Methylenchlorid bei -30°C vorgelegt. 275 mmol Benzylalkohol werden so zugetropft, daß die Temperatur der Reaktionslösung 5°C nicht übersteigt. Nach anschließendem eineinhalbstündigem Rühren bei 0°C wird eine Suspension von 302 mmol Pyrrolidin-2-carbon säuremethylester-Hydrochlorid und 815 mmol Triethylamin in 1 l trockenem Methylenchlorid ebenfalls so zugetropft, daß 5°C im Ansatz nicht überschritten werden. Die Reaktionslösung wird nach Rühren über Nacht bei Raumtemperatur mehrmals mit insgesamt 350 ml 2n Salzsäure extrahiert, mit Kochsalzlösung gewaschen, getrocknet und einrotiert. Die Titelverbindung wird in ca. 90 %iger Ausbeute als Öl erhaltenen.
$^1$H-NMR ($CDCl_3$): $\delta$ = 1.66 - 2.40 (m, 4H, 3-$CH_2$, 4-$CH_2$), 3.31 - 3.86 (m, 2H, 5-$CH_2$), 3.71 (s, 3H, $CH_3$), 4.64 (dd, $J_1$ = 7 Hz, $J_2$ = 3 Hz, 1H, 2-CH), 5.17 (s, 2H, $CH_2$-Ph), 7.35 (s, 5H, Ph-H), 7.84 (s-breit, 1H, NH).
IR (Ölfilm): 2960 (breit), 1740, 1650, 1600, 1450, 1360, 1340, 1285, 1215, 1155, 1100, 1080, 1040, 1025, 845, 750, 700 $cm^{-1}$.

**Stufe C:**

**N-Sulfamoylpyrrolidin-2-carbonsäuremethylester**

228 mmol N-[N'-(Benzyloxycarbonyl)sulfamoyl]pyrrolidin-2-carbonsäuremethylester werden in 200 ml Methanol über Palladium/Aktivkohle bei Raumtemperatur hydriert. Die Zielverbindung fällt während der Hydrierung im Reaktionsgefäß aus. Der katalysatorhaltige Niederschlag wird abgesaugt und mehrmals mit Aceton aufgekocht, wobei das Produkt in Lösung geht. Nach heisser Filtration und Abziehen des Lösungsmittels erhält man in durchschnittlich 76 %iger Ausbeute die Titelverbindung als kristallines Rohprodukt, das mit Essigester gewaschen wird.
Schmp.: 166 - 167.5°C
$^1$H-NMR ($d_6$-DMSO): $\delta$ = 1.61 - 2.30 (m, 4H, 3-$CH_2$, 4-$CH_2$), 3.26 (t, J = 6 Hz, 2H, 5-$CH_2$), 3.61 (s, 3H, $CH_3$), 4.17 (dd, $J_1$ = 8 Hz, $J_2$ = 3 Hz, 1H, 5-CH), 6.81 (s, 2H, $NH_2$).
IR (KBr): 3340, 3240, 3100, 2980, 2955, 2870, 2675, 2495, 1735, 1655, 1560, 1460, 1440, 1385, 1360, 1325, 1280, 1220, 1200, 1185, 1165, 1150, 1135, 1100, 1070, 1015, 990, 910, 830, 760 $cm^{-1}$.

**Stufe D:**

**1,1-Dioxo-pyrrolidino[4,5-d][1,2,5]thiadiazolidin-3-on**

377 mmol N-Sulfamoylpyrrolidin-2-carbonsäuremethylester werden in einer frisch zubereiteten Lösung von 1.4 eq Natriummethanolat in Methanol drei Stunden unter Rückfluß erhitzt. Abziehen des Lösemittels, Aufnehmen des Rückstandes in 0.3 l Essigsäureethylester, mehrmalige Extraktion der organischen Lösung mit 2n Salzsäure, Waschen mit Kochsalzlösung, Trocknen und Abziehen des Lösungsmittels liefert in etwa 40 %iger Ausbeute die farblose, kristalline Titelverbindung.
Schmp.: 134 - 135°C
$^1$H-NMR ($d_6$-DMSO): $\delta$ = 1.48 - 2.41 (m, 4H, 4-$CH_2$,5-$CH_2$), 3.00 - 3.83 (m, 2H, 6-$CH_2$), 4.56 (dd, $J_1$ = 7 Hz, $J_2$ = 1 Hz, 1H, 3a-CH), 9.58 (s-breit, 1H, NH).
IR (KBr): 3130 (breit), 2670, 1635, 1615, 1460, 1365, 1335, 1300, 1265, 1180, 1090, 1070, 990, 975, 965, 930, 925, 885, 700 $cm^{-1}$.
MS: m/e = 176 ($M^{+\cdot}$), 148, 133, 106, 79, 69 (100 %), 68, 64, 48.

| Elementaranalyse: ($C_5H_8N_2O_3S$) | | | | |
|---|---|---|---|---|
| | % C | % H | % N | % S |
| berechnet: | 34.08 | 4.58 | 15.90 | 18.20 |
| gefunden: | 34.47 | 4.57 | 15.42 | 17.55 |

**Stufe E:**

**2-(1',1'-Dioxo-pyrrolidino[4,5-d][1,2,5]thiadiazolidin-3'-on-2'-yl)essigsäuremethylester**

329 mmol 1,1-Dioxo-pyrrolidino[4,5-d][1,2,5]thiadiazolidin-3-on werden in 400 ml trockenem THF gelöst und mit 427 mmol Triethylamin und 395 mmol Bromessigsäuremethylester drei Stunden unter Rückfluß erhitzt. Das Ammoniumsalz wird abgesaugt und nachgewaschen, die organische Lösung weitgehend einrotiert, der Rückstand in Essigsäureethylester aufgenommen und mehrmals zuerst mit 2n Salzsäure, dann mit Kochsalzlösung extrahiert, getrocknet und einrotiert. Das zunächst ölige Rohprodukt kristallisiert beim Stehenlassen weitgehend aus. Die verbleibende ölige Mutterlauge wird mit Diisopropylether/Essigsäureethylester/Ameisensäure = 100/50/1 über Kieselgel chromatographiert. Waschen der vereinigten Kristalle mit tert.-Butylmethylether liefert die Titelverbindung mit durchschnittlich 79 %iger Ausbeute.
Schmp.: 63 - 63.5 °C
$^1$H-NMR ($CDCl_3$): $\delta$ = 1.69 - 2.61 (m, 4H, 4'-$CH_2$, 5'-$CH_2$), 3.12 - 4.08 (m, 2H, 6'-$CH_2$), 3.79 (s, 3H, $CH_3$), 4.25 (d, J = 2 Hz, 2H, 2-$CH_2$), 4.53 (dd, $J_1$ = 7 Hz, $J_2$ = 3 Hz, 1H, 3a-CH).
IR (KBr): 3490, 3460, 2980, 2960, 2880, 1750, 1445, 1410, 1380, 1360, 1320, 1305, 1295, 1250, 1200, 1110, 1065, 1040, 990, 970, 950, 925, 905, 885, 870, 815, 760, 715, 660, 630 $cm^{-1}$.

**Stufe F:**

**5,6-Dihydro-4-hydroxy-1,1-dioxo-2H-pyrrolidino[1,2-e][1,2,6]thiadiazin-3-carbonsäuremethylester**

332 mmol Natrium werden in 200 ml absolutem Methanol gelöst. Das nach Abziehen des Lösungsmittels erhaltene Natriummethanolat wird in trockenem DMSO aufgeschlämmt und im Eisbad gekühlt. Eine ebenfalls vorgekühlte Lösung aus 166 mmol 2-(1',1'-Dioxopyrrolidino[4,5-d][1,2,5]thiadiazolidin-3'-on-2'-yl) essigsäuremethylester in DMSO wird rasch zugetropft. Nach beendeter Zugabe rührt man weitere 15 Minuten und versetzt dann mit 200 ml einer Salzsäure-Eis-Mischung. Dann wird die Mischung mit Essigsäureethylester, die Essigesterphase mehrmals mit 2n Natronlauge extrahiert. Ansäuern der wäßrig alkalischen Lösung mit 2n Salzsäure, Rückextraktion mit Essigsäureethylester, Waschen der organischen Lösung mit Kochsalzlösung, Trocknen und Abziehen des Lösungsmittels liefert ein öliges Rohprodukt, das nach Versetzen mit tert.-Butylmethylether auskristallisiert. Man erhält so in durchschnittlich 20 bis 30 %iger Ausbeute die saubere Titelverbindung.
Schmp.: 109.5 - 110 °C
$^1$H-NMR ($CDCl_3$): $\delta$ = 1.73 - 2.53 (m, 4H, 5-$CH_2$, 6-$CH_2$), 2.91 - 4.66 (m, 2H, 7-$CH_2$), 3.83 (s, 3H, $CH_3$), 4.54 (t, J = 5 Hz, 1H, 4a-CH), 5.91 (s-breit, 1H, NH), 11.15 (s, 1H, OH).
IR (KBr): 3280, 2950, 2890, 1670, 1620, 1450, 1410, 1380, 1310, 1250, 1225, 1175, 1155, 1110, 1090, 1060, 1040, 985, 895, 780 $cm^{-1}$.
MS: m/e = 248 ($M^{+\cdot}$), 216, 148, 124, 99, 87, 70 (100 %), 68, 55.

**Stufe G:**

**5,6-Dihydro-4-hydroxy-2-methyl-1,1-dioxo-2H-pyrrolidino[1,2-e][1,2,6]thiadiazin-3-carbonsäuremethylester**

Zu einer Lösung von 86 mmol 5,6-Dihydro-4-hydroxy-1,1-dioxo-2H-pyrrolidino[1,2-e][1,2,6]thiadiazin-3-carbonsäuremethylester in 350 ml trockenem THF tropft man bei -30 °C 2.5 eq einer 2.5 molaren Lösung von Butyllithium in Hexan so zu, daß die Reaktionstemperatur -25 °C nicht übersteigt. Nach beendeter Zugabe läßt man 20 Minuten bei tiefer Temperatur rühren, und tropft anschließend, ebenfalls bei -30 °C 3.0 eq Methyliodid in trockenem THF dazu. Nach zwanzigminütigem Rühren bei tiefer Temperatur läßt man

den Ansatz auf Raumtemperatur erwärmen und rührt weitere 15 Stunden. Schwaches Ansäuern mit verdünnter Salzsäure, weitgehendes Abziehen des Lösungsmittels im Vakuum, Aufnehmen des Rückstandes in Essigsäureethylester, Waschen der Essigesterlösung mit Kochsalzlösung, Trocknen und zuletzt Abziehen des Lösungsmittels liefert ein rotbraunes, öliges Rohprodukt, das mit einem Lösungsmittelgemisch aus Diisopropylether/Essigsäureethylester/Ameisensäure = 100/50/1 über Kieselgel chromatographiert wird. Die ölige Produktfraktion kristallisiert nach Versetzen mit tert.-Butylmethylether blaßgelb in durchschnittlich 46 %iger Ausbeute zur Titelverbindung aus.
Schmp.: 89.5 - 90.5 °C
$^1$H-NMR ($CDCl_3$): $\delta$ = 1.73 - 2.58 (m, 4H, 5-$CH_2$, 6-$CH_2$), 3.10 (s, 3H, N-$CH_3$), 3.20 - 3.96 (m, 2H, 7-$CH_2$), 3.86 (s, 3H, O-$CH_3$), 4.35 (t, J = 7 Hz, 1H, 4a-CH), 11.83 (s, 1H, OH).
IR (KBr): 2980, 2950, 1665, 1605, 1440, 1365, 1335, 1245, 1210, 1155, 1060, 1035, 995, 965, 855, 820, 805, 780, 765, 700, 680 $cm^{-1}$.

**Beispiel 8:**

**2-Ethyl-5,6-dihydro-4-hydroxy-1,1-dioxo-N-phenyl-2H-pyrrolidino[1,2-e][1,2,6]thiadiazin-3-carbonsäureamid**

Die Darstellung erfolgt analog Beispiel 1 mit je 1.0 eq 2-Ethyl-5,6-dihydro-4-hydroxy-1,1-dioxo-2H-pyrrolidino[1,2-e][1,2,6]thiadiazin-3-carbonsäuremethylester und Anilin. Nach Umkristallisation aus einer Diisopropylether/Aceton-Mischung wird die Titelverbindung in 35 %iger Ausbeute erhalten.
Schmp.: 132 - 133 °C
$^1$H-NMR ($CDCl_3$): $\delta$ = 1.31 (t, J = 7 Hz, 3H, $CH_3$) 1.66 - 2.58 (m, 4H, 5-$CH_2$ und 6-$CH_2$), 3.13 - 3.97 (m, 4H, 7-$CH_2$ und N-$CH_2$), 4.28 (dd, $J_1$ = 7 Hz, $J_2$ = 12 Hz, 1H, 4a-CH), 7.00 - 7.73 (m, 5H, Ph-H), 8.08 (s-breit, 1H, NH).
IR (KBr): 3300, 2970, 2870, 1630, 1595, 1535, 1495, 1445, 1325, 1230, 1165, 1090, 1060, 1040, 1030, 990, 955, 930, 900, 890, 855, 795, 745, 685 $cm^{-1}$.
MS: m/e = 337 ($M^{+\cdot}$), 218, 161, 147, 120, 93 (100 %), 92, 77, 70, 69, 68, 56.

Die in **Beispiel 8** als Ausgangsprodukt verwendete Verbindung 2-Ethyl-5,6-dihydro-4-hydroxy-1,1-dioxo-2H-pyrrolidino[1,2-e][1,2,6]thiadiazin-3-carbonsäuremethylester wurde analog zu den Stufen A bis G unter Verwendung von Ethyliodid anstelle von Methyliodid in Stufe G erhalten.

**Beispiel 9:**

**2-Benzyl-5,6-dihydro-4-hydroxy-1,1-dioxo-N-phenyl-2H-pyrrolidino[1,2-e][1,2,6]thiadiazin-3-carbonsäureamid**

Die Darstellung erfolgt analog Beispiel 1 mit je 1.0 eq 2-Benzyl-5,6-dihydro-4-hydroxy-1,1-dioxo-2H-pyrrolidino[1,2-e][1,2,6]thiadiazin-3-carbonsäuremethylester und Anilin. Nach Umkristallisation aus einer Diisopropylether/Aceton-Mischung wird die Titelverbindung in 21 %iger Ausbeute erhalten.
Schmp.: 147 - 149 °C
$^1$H-NMR ($CDCl_3$): $\delta$ = 1.66 - 2.56 (m, 4H, 5-$CH_2$ und 6-$CH_2$), 3.20 - 3.91 (m, 2H, 7-$CH_2$), 3.97 - 4.28 (m, 1H, 4a-CH), 4.59 (s-breit, 2H, $CH_2$-Ph), 6.87 - 7.64 (m, 11H, 2 x 5 Ph-H und 1 NH).
IR (KBr): 3360, 3310, 2920, 2860, 1635, 1595, 1535, 1490, 1445, 1420, 1405, 1330, 1315, 1230, 1215, 1160, 1035, 1025, 975, 920, 890, 850, 805, 790 $cm^{-1}$.
MS: m/e = 399 ($M^{+\cdot}$), 308, 280, 238, 147, 120, 93, 92, 91, 77, 70 (100 %), 69, 68, 65.

Die in **Beispiel 9** als Ausgangsprodukt verwendete Verbindung 2-Benzyl-5,6-dihydro-4-hydroxy-1,1-dioxo-2H-pyrrolidino[1,2-e][1,2,6]thiadiazin-3-carbonsäuremethylester wurde analog zu den Stufen A bis G unter Verwendung von Benzylbromid anstelle von Methyliodid in Stufe G erhalten.

**Beispiel 10:**

**5,6-Dihydro-4-hydroxy-2-methyl-1,1-dioxo-N-phenyl-2H-piperidino[1,2-e][1,2,6]thiadiazin-3-carbonsäureamid**

Die Darstellung erfolgt analog Beispiel 1 mit je 1 eq 5,6-Dihydro-4-hydroxy-2-methyl-1,1-dioxo-2H-piperidino[1,2-e][1,2,6]thiadiazin-3-carbonsäuremethylester und Anilin. Nach säulenchromatographischer Aufreinigung über Kieselgel wird die Titelverbindung in 50 %iger Ausbeute erhalten.

Schmp.: 214 - 215 °C, unter Zersetzung
$^1$H-NMR ($d_6$-DMSO): δ = 1.22 - 3.00 (m, 8H, 5-$CH_2$, 6-$CH_2$, 7-$CH_2$ und 8-$CH_2$), 2.90 (s, 3H, N-$CH_3$), 3.44 - 3.80 (m, 1H, 4a-CH), 6.96 - 7.76 (m, 5H, Ph-H), 9.92 (s, 1H, NH), 13.76 (s-breit, 1H, OH).
IR (KBr): 3330, 2990, 2950, 2920, 2850, 1635, 1595, 1530, 1500, 1445, 1355, 1340, 1325, 1295, 1260, 1225, 1210, 1180, 1170, 1140, 1105, 1080, 1065, 1050, 1035, 1005, 995, 955, 940, 925, 905, 895, 875, 820, 805, 770, 750, 740, 700, 635 $cm^{-1}$.
MS: m/e = 337 ($M^{+\cdot}$), 176, 161, 134, 133, 120, 93 (100 %), 92, 84, 83, 77, 66, 65, 56, 55.

| Elementaranalyse: ($C_{15}H_{19}N_3O_4S$) | | | | |
|---|---|---|---|---|
| | % C | % H | % N | % S |
| berechnet: | 53.38 | 5.67 | 12.46 | 9.50 |
| gefunden: | 53.52 | 5.21 | 12.40 | 9.50 |

Die in **Beispiel 10** als Ausgangsprodukt verwendete Verbindung 5,6-Dihydro-4-hydroxy-2-methyl-1,1-dioxo-2H-piperidino[1,2-e][1,2,6]thiadiazin-3-carbonsäuremethylester wurde analog zu den Stufen A bis G unter Verwendung von Piperidin-2-carbonsäure anstelle von Prolin in Stufe A erhalten.

## Beispiel 11:

### 6-Benzyl-5,6-dihydro-4-hydroxy-2-methyl-1,1-dioxo-N-phenyl-2H-1,2,6-thiadiazin-3-carbonsäureamid

Die Darstellung erfolgt analog Beispiel 1 mit je 1 eq 6-Benzyl-5,6-dihydro-4-hydroxy-2-methyl-1,1-dioxo-2H-1,2,6-thiadiazin-3-carbonsäuremethylester und Anilin. Die Titelverbindung wird nach Umkristallisation aus einer Diisopropylether-Aceton-Mischung in 54 %iger Ausbeute erhalten.
Schmp.: 197 - 198 °C
$^1$H-NMR ($d_6$-DMSO): δ = 2.96 (s, 3H, $CH_3$), 3.79 (s, 2H, 5-$CH_2$), 4.33 (s, 2H, Ph-$CH_2$), 7.00 - 7.79 (m, 10H, 2 x 5 Ph-H), 9.89 (s, 1H, NH).
IR (KBr): 3350, 1640, 1595, 1540, 1490, 1445, 1430, 1395, 1345, 1330, 1290, 1250, 1205, 1165, 1145, 1120, 1090, 1065, 1040, 1110, 975, 935, 910, 885, 845, 825, 815, 770, 760, 730, 700, 660, 640 $cm^{-1}$.
MS: m/e = 373 ($M^{+\cdot}$), 254, 190, 173, 161, 147, 133, 120, 106, 93 (100 %), 77, 69, 65.

| Elementaranalyse: ($C_{18}H_{19}N_3O_4S$) | | | | |
|---|---|---|---|---|
| | % C | % H | % N | % S |
| berechnet: | 57.90 | 5.13 | 11.25 | 8.59 |
| gefunden: | 57.89 | 5.26 | 11.18 | 8.45 |

## Beispiel 12:

### 6-Benzyl-5,6-dihydro-4-hydroxy-2-methyl-N-(5-methyl-3-isoxazolyl)-1,1-dioxo-2H-1,2,6-thiadiazin-3-carbonsäureamid

Die Darstellung erfolgt analog Beispiel 1 mit je 1 eq 6-Benzyl-5,6-dihydro-4-hydroxy-2-methyl-1,1-dioxo-2H-1,2,6-thiadiazin-3-carbonsäuremethylester und 3-Amino-5-methylisoxazol. Nach Chromatographie über Kieselgel mit einer Mischung aus Diisopropylether/Ameisensäure = 100/1 bzw. Umkristallisation aus einer Diisopropylether/Aceton-Mischung wird die Titelverbindung in 59 %iger Ausbeute erhalten.
Schmp.: 199 - 199.5 °C
$^1$H-NMR ($d_6$-DMSO): δ = 2.38 (s, 3H, Ar-$CH_3$), 2.92 (s, 3H, N-$CH_3$), 3.81 ( s, 2H, 5-$CH_2$), 4.33 (s, 2H, Ph-$CH_2$), 6.59 (s, 1H, Ar-H), 7.40 (s, 5H, Ph-H), 10.83 (s, 1H, NH).
IR (KBr): 3200, 1650, 1610, 1545, 1535, 1470, 1450, 1435, 1380, 1355, 1330, 1275, 1250, 1220, 1170, 1165, 1125, 1085, 1055, 1030, 1010, 990, 955, 935, 935, 900, 885, 860, 830, 810, 785, 755, 735, 695, 655, 620 $cm^{-1}$.

| Elementaranalyse: ($C_{16}H_{18}N_4O_5S$) | | | | |
|---|---|---|---|---|
| | % C | % H | % N | % S |
| berechnet: | 50.79 | 4.79 | 14.81 | 8.47 |
| gefunden: | 50.66 | 4.67 | 14.71 | 8.56 |

Die in den **Beispielen 11** und **12** als Ausgangsprodukt verwendete Verbindung 6-Benzyl-5,6-dihydro-4-hydroxy-2-methyl-1,1-dioxo-2H-1,2,6-thiadiazin-3-carbonsäuremethylester wurde analog zu den Stufen A bis G unter Verwendung von N-Benzylglycin anstelle von Prolin in Stufe A erhalten.

**Beispiel 13:**

**5,6-Dihydro-4-hydroxy-2,6-dimethyl-N-(2-pyridyl)-1,1-dioxo-2H-1,2,6-thiadiazin-3-carbonsäureamid**

Die Darstellung erfolgte analog Beispiel 1 mit je 1.0 eq 5,6-Dihydro-4-hydroxy-2,6-dimethyl-1,1-dioxo-2H-1,2,6-thiadiazin-3-carbonsäuremethylester und 2-Aminopyridin. Nach Umkristallisation aus einer Diisopropylether/Tetrahydrofuran-Mischung wird die Titelverbindung in 55 %iger Ausbeute erhalten.
Schmp.: 185 °C, unter Zersetzung
$^1$H-NMR ($d_6$-DMSO): $\delta$ = 2.73 (s, 3H, 2-N-$CH_3$), 2.92 (s, 3H, 6-N-$CH_3$), 3.86 ( s, 2H, 5-$CH_2$), 7.08 - 7.35 (m, 1H, 1 Ar-H), 7.68 - 8.07 (m, 2H, 2 Ar-H), 8.26 - 8.46 (m, 1H, 1 Ar-H), 10.83 (s-breit, 1H, NH).
IR (KBr): 3360, 2940, 1660, 1605, 1590, 1575, 1525, 1460, 1430, 1380, 1350, 1345, 1325, 1300, 1240, 1210, 1175, 1160, 1125, 1115, 1050, 1010, 985, 970, 895, 885, 845, 830, 820, 790, 760, 730, 680, 630, 620, 610 $cm^{-1}$.
NS: m/e = 298 ($M^{+\cdot}$), 219, 203, 191, 163, 148, 134, 121, 94 (100 %), 78, 67, 51.

**Beispiel 14:**

**5,6-Dihydro-4-hydroxy-2,6-dimethyl-N-(5-methyl-3-isoxazolyl)1,1-dioxo-2H-1,2,6-thiadiazin-3-carbonsäureamid**

Die Darstellung erfolgte analog Beispiel 1 mit je 1.0 eq 5,6-Dihydro-4-hydroxy-2,6-dimethyl-1,1-dioxo-2H-1,2,6-thiadiazin-3-carbonsäuremethylester und 3-Amino-5-methylisoxazol. Nach Umkristallisation aus einer Diisopropylether/Tetrahydrofuran-Mischung wird die Titelverbindung in 49 %iger Ausbeute erhalten.
Schmp.: 189 - 191.5 °C, unter Zersetzung
$^1$H-NMR ($d_6$-DMSO): $\delta$ = 2.41 (s, 3H, Ar-$CH_3$), 2.73 (s, 3H, 2-N-$CH_3$), 2.89 (s, 3H, 6-N-$CH_3$), 3.94 (s, 2H, 5-$CH_2$), 6.59 (s, 1H, Ar-H), 10.83 (s, 1H, NH).
IR (KBr): 3195, 2930, 1645, 1610, 1535, 1470, 1445, 1435, 1400, 1375, 1355, 1340, 1330, 1300, 1275, 1255, 1220, 1200, 1170, 1145, 1125, 1115, 1060, 1030, 1005, 990, 975, 935, 885, 835, 785, 770, 760, 730, 630, 620 $cm^{-1}$.
MS: m/e = 302 ($M^{+\cdot}$), 207, 195, 180, 167, 152 (100 %), 138, 125, 111, 98, 83, 69, 57, 55.

Die in den Beispielen **13** und **14** als Ausgangsprodukt verwendete Verbindung 5,6-Dihydro-4-hydroxy-2,6-dimethyl-1,1-dioxo-2H-1,2,6-thiadiazin-3-carbonsäuremethylester wurde analog zu den Stufen A bis G unter Verwendung von Sarcosin anstelle von Prolin in Stufe A erhalten.

**Beispiel 15:**

Beim Vorgehen analog Beispiel 1 unter Verwendung der nachstehend dargestellten Edukte gemäß den allgemeinen Formeln **II** und **III** erhält man die betreffenden erfindungsgemäßen Verbindungen **I** der nachstehenden Tabelle (Beispiele 15a - 15l):

| Edukt Formel II | Edukt Formel III | Produkt Formel I | Beisp.; Schmp. |
|---|---|---|---|
| 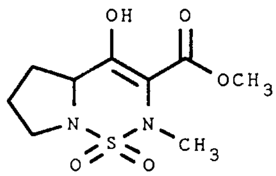 | 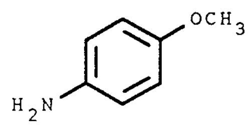 | 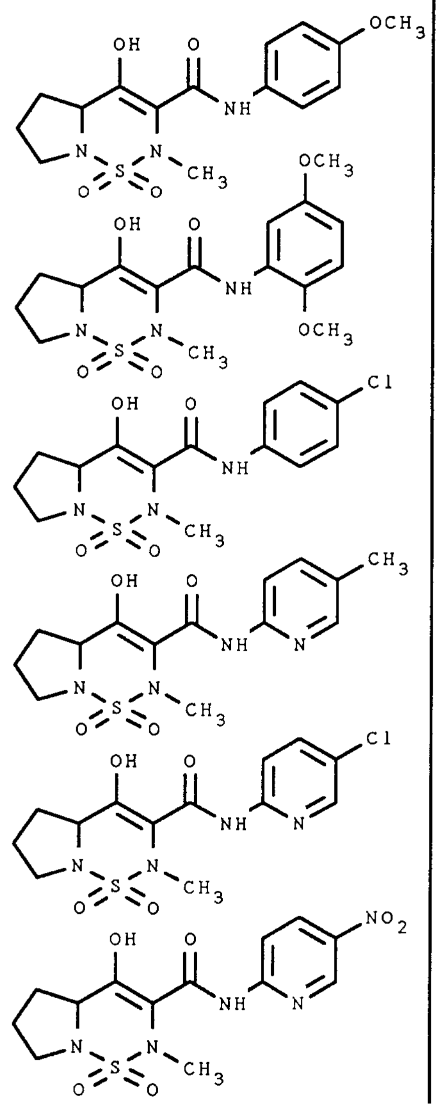 | 15a; 189-190°C |
| " | 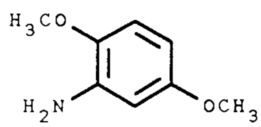 | | 15b; 153-154°C |
| " | 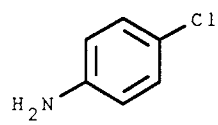 | | 15c; 188-189°C |
| " | 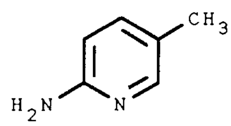 | | 15d; 162-163°C |
| " | 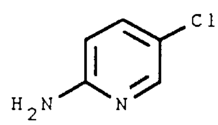 | | 15e; 155-156°C |
| " | 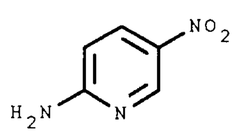 | | 15f; 202-204°C |

| Edukt Formel II | Edukt Formel III | Produkt Formel I | Beisp.; Schmp. |
|---|---|---|---|
| 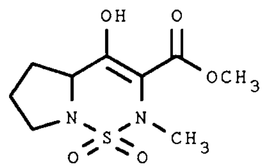 | 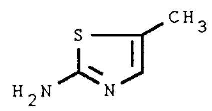 | 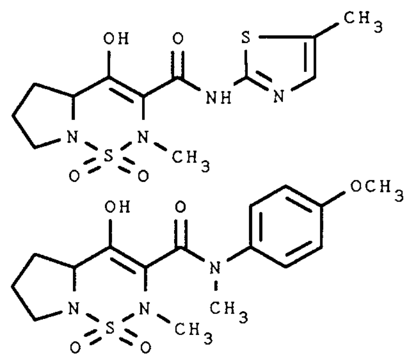 | 15g; 190°C (Zers.) |
| " | 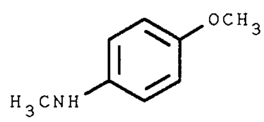 | | 15h; 131-132°C |
| 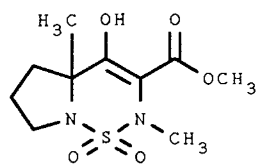 | 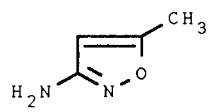 | 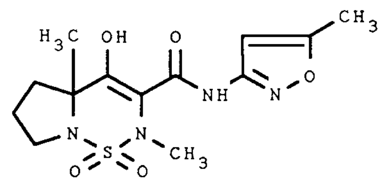 | 15i; 216-217°C |
| 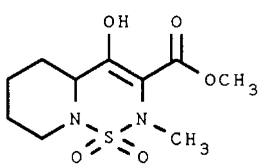 | 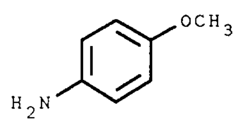 | 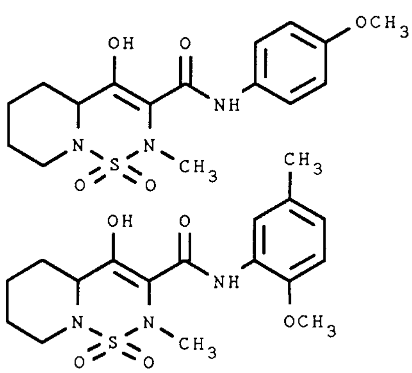 | 15j; 198-199°C |
| " | 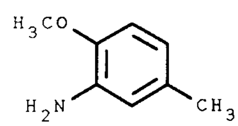 | | 15k; 210-211°C |
| 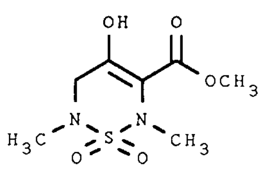 | 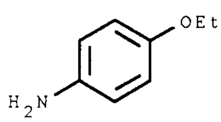 | 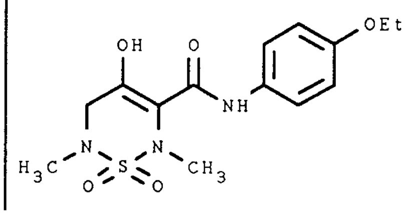 | 15l; 173-174°C |

**Beispiel 16:**

**Rezeptur zur Herstellung von Tabletten:**

1000 Tabletten stellt man aus den nachstehenden Verbindungen auf die unten beschriebene Weise her. Eine Tablette enthält dann als Wirkstoff 100 mg 5,6-Dihydro-4-hydroxy-2-methyl-1,1-dioxo-N-(2-pyridyl)-2H-pyrrolidino[1,2-e][1,2,6]thiadiazin-3-carbonsäureamid.

| | |
|---|---|
| 1. 5,6-Dihydro-4-hydroxy-2-methyl-1,1-dioxo-N- (2-pyridyl)-2H-pyrrolidino[1,2-e][1,2,6]thiadiazin-3-carbonsäureamid | 100 g |
| 2. Milchzucker | 263 g |
| 3. mikrokristalline Cellulose | 120 g |
| 4. Maisstärke | 60 g |
| 5. Magnesiumstearat | 7 g |

Man mischt 1) und 2), mischt 3) und 4) unter, versetzt zuletzt mit 5), mischt und verpreßt direkt.

**Beispiel 17:**

**Rezeptur zur Herstellung einer Creme:**

Die folgende Rezeptur ergibt eine 5 %ige 5,6-Dihydro-4-hydroxy-2-methyl-1,1-dioxo-N-(2-pyridyl)-2H-pyrrolidino[1,2-e][1,2,6]thiadiazin-3-carbonsäureamid-Creme (Stoffangaben in Gew.-%):

| | |
|---|---|
| 5,6-Dihydro-4-hydroxy-2-methyl-1,1-dioxo-N-(2-pyridyl)-2H-pyrrolidino[1,2-e][1,2,-6]thiadiazin-3-carbonsäureamid | 5,00 |
| Emulgator (Gemisch aus Natriumglycerylmonostearat, Natriumstearylsulfat und Natriumcetylsulfat) | 10,00 |
| Mittelkettige Triglyceride | 6,25 |
| Myristylalkohol | 5,00 |
| POE-12-Cetylstearylalkohol | 3,00 |
| Konservierungsmittel | q.s. |
| Wasser | ad 100,00 |

## Patentansprüche

**1.** Verbindungen der allgemeinen Formel **I**

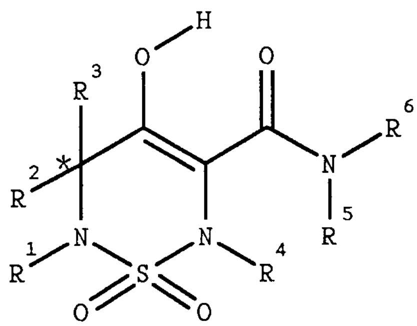

(I)

worin

**R**$^1$ einen Niedrigalkylrest, einen Arylrest, einen Heteroarylrest oder einen Arylniedrigalkylrest bedeutet,

**R**$^2$ ein Wasserstoffatom, einen Niedrigalkylrest oder einen Arylrest bedeutet,

**R**$^3$ ein Wasserstoffatom, einen Niedrigalkylrest, einen Arylrest, einen Heteroarylrest, einen Arylniedrigalkylrest oder einen gesättigten, unverzweigten $C_1$-$C_4$-Alkylrest bedeutet, der mit einem Rest aus der Gruppe **-OR**$^7$, **-NR**$^8$**R**$^9$, **-CO-OR**$^{10}$, **-SR**$^{11}$, **-CO-NR**$^{12}$**R**$^{13}$ oder **-NH-C(NH**$_2$**)(=NH)** einfach substituiert ist,

oder

worin **R**$^1$ und **R**$^3$ zusammen einen unverzweigten, gesättigten Alkylenrest mit zwei, drei, vier oder fünf Kohlenstoffatomen bilden und so mit dem benachbarten Stickstoffatom und Kohlenstoffatom des Thiadiazinringsystems einen Ring mit vier, fünf, sechs oder sieben Ringgliedern bilden,

**R**$^4$ ein Wasserstoffatom, einen Niedrigalkylrest oder einen Arylniedrigalkylrest bedeutet,

**R**$^5$ ein Wasserstoffatom oder einen Niedrigalkylrest bedeutet,

**R**$^6$ ein Wasserstoffatom, einen Niedrigalkylrest, einen Arylrest, einen Heteroarylrest oder einen Aryl-

niedrigalkylrest bedeutet,
wobei **R**$^{7}$, **R**$^{8}$, **R**$^{9}$, **R**$^{10}$, **R**$^{11}$, **R**$^{12}$ und **R**$^{13}$ unabhängig voneinander ein Wasserstoffatom, einen Niedrigalkylrest, einen Arylrest oder einen Arylniedrigalkylrest bedeuten,
und deren Salze mit physiologisch verträglichen Säuren und Basen,
wobei der vorstehend genannte Ausdruck Arylrest einen Phenylrest, einen 1-Naphtylrest oder einen 2-Naphtylrest bedeutet, der gegebenenfalls mit ein, zwei oder drei gleichen oder verschiedenen Substituenten aus der Gruppe der Halogenatome, Hydroxylreste, Niedrigalkylreste, Niedrigalkyloxyreste, Carbonsäurereste, Niedrigalkyloxycarbonylreste, Nitrogruppen, Sulfonsäurereste, Trifluormethylreste oder mit Wasserstoffatomen oder Niedrigalkylresten substituierten Aminogruppen substituiert ist,
und wobei
der vorstehend genannte Ausdruck Heteroarylrest einen der folgenden Reste bedeutet: Furanylreste, Thienylreste, Pyrrolylreste, Pyrazolylreste, Imidazolylreste, Triazolylreste, Thiazolylreste, Oxazolylreste, Isothiazolylreste, Isoxazolylreste, Thiadiazolylreste, Pyridylreste, Pyrimidylreste, Pyrazinylreste, Triazinylreste, Benzofuranylreste, Benzothienylreste, Indolylreste, Benzoxazolylreste, Benzothiazolylreste, Benzimidazolylreste, Chinolinylreste oder Isochinolinylreste, wobei vorstehend genannte Reste über jedes Ringkohlenstoffatom mit dem Grundgerüst der Verbindungen der allgemeinen Formel I verknüpft sein können und wobei die vorstehend genannten Ringe gegebenenfalls mit ein oder zwei gleichen oder verschiedenen Substituenten aus der Gruppe der Halogenatome, Hydroxylreste, Niedrigalkylreste, Niedrigalkyloxyreste, Carbonsäurereste, Niedrigalkyloxycarbonylreste, Nitrogruppen, Sulfonsäurereste, Trifluormethylreste oder mit Wasserstoffatomen oder Niedrigalkylresten substituierten Aminogruppen substituiert sein können,
und wobei
der vorstehend genannte Ausdruck Arylniedrigalkylrest einen Methylrest oder einen Ethylrest bedeutet, der mit einem Arylrest substituiert ist, der wie obenstehend definiert ist,
und wobei
der vorstehend genannte Ausdruck Niedrigalkylrest bzw. der Ausdruck "Niedrigalkyl" im Zusammenhang mit Niedrigalkyloxyrest oder Niedrigalkyloxycarbonylrest einen unverzweigten oder verzweigten gesättigten Kohlenwasserstoffrest mit bis zu sechs Kohlenstoffatomen bedeutet.

**2.** Verbindungen gemäß Anspruch **1**, worin **R**$^{2}$ ein Wasserstoffatom bedeutet.

**3.** Verbindungen gemäß Anspruch **2**, worin **R**$^{1}$ und **R**$^{3}$ zusammen einen unverzweigten, gesättigten Alkylenrest mit zwei, drei, vier oder fünf Kohlenstoffatomen bilden und so mit dem benachbarten Stickstoffatom und Kohlenstoffatom des Thiadiazinringsystems einen Ring mit vier, fünf, sechs oder sieben Ringgliedern bilden.

**4.** Verbindungen gemäß Anspruch **2**, worin **R**$^{6}$ einen wie vorstehend definierten Arylrest bedeutet.

**5.** Verbindungen gemäß Anspruch **2**, worin **R**$^{6}$ einen wie vorstehend definierten Heteroarylrest bedeutet.

**6.** Verbindungen gemäß Anspruch **3**, worin **R**$^{1}$ und **R**$^{3}$ zusammen einen Propylenrest bedeuten und so mit dem benachbarten Stickstoffatom und Kohlenstoffatom des Thiadiazinringsystems einen Pyrrolidinring bilden.

**7.** Verbindungen gemäß Anspruch **6**, worin **R**$^{6}$ einen wie vorstehend definierten Arylrest bedeutet.

**8.** Verbindungen gemäß Anspruch **6**, worin **R**$^{6}$ einen wie vorstehend definierten Heteroarylrest bedeutet.

**9.** Verbindungen gemäß Anspruch **4**, worin **R**$^{6}$ eine der Bedeutungen Phenylrest, 2-Methylphenylrest, 4-Methylphenylrest, 2-Methoxyphenylrest, 4-Methoxyphenylrest, 2-Methoxy-5-methylrest, 2,3-Dimethoxyphenylrest, 2,4-Dimethoxyphenylrest, 2,5-Dimethoxyphenylrest, 2,6-Dimethoxyphenylrest, 3,4-Dimethoxyphenylrest, 3,5-Dimethoxyphenylrest, 2-Ethoxyphenylrest, 4-Ethoxyphenylrest, 2-Chlorphenylrest, 4-Chlorphenylrest, 2,4-Dichlorphenylrest, 2,6-Dichlorphenylrest, 2-Fluorphenylrest, 4-Fluorphenylrest oder 3-Trifluormethylphenylrest hat.

**10.** Verbindungen gemäß Anspruch **5**, worin **R**$^{6}$ einen 2-Pyridylrest, einen 2-Thiazolylrest, einen 5-Methyl-2-thiazolylrest oder einen 5-Methyl-3-isoxazolylrest bedeutet.

**11.** Verbindungen gemäß Anspruch **8**, worin $\mathbf{R}^6$ einen 2-Pyridylrest, einen 2-Thiazolylrest, einen 5-Methyl-2-thiazolylrest oder einen 5-Methyl-3-isoxazolylrest bedeutet.

**12.** Verbindungen gemäß Anspruch **7**, worin $\mathbf{R}^6$ eine der Bedeutungen Phenylrest, 2-Methylphenylrest, 4-Methylphenylrest, 2-Methoxyphenylrest, 4-Methoxyphenylrest, 2-Methoxy-5-methylrest, 2,3-Dimethoxyphenylrest, 2,4-Dimethoxyphenylrest, 2,5-Dimethoxyphenylrest, 2,6-Dimethoxyphenylrest, 3,4-Dimethoxyphenylrest, 3,5-Dimethoxyphenylrest, 2-Ethoxyphenylrest, 4-Ethoxyphenylrest, 2-Chlorphenylrest, 4-Chlorphenylrest, 2,4-Dichlorphenylrest, 2,6-Dichlorphenylrest, 2-Fluorphenylrest, 4-Fluorphenylrest oder 3-Trifluormethylphenylrest hat.

**13.** Verfahren zur Herstellung von Verbindungen der allgemeinen Formel **I** gemäß Anspruch **1**, dadurch gekennzeichnet, daß man in an sich bekannter Weise eine Verbindung der allgemeinen Formel **II**,

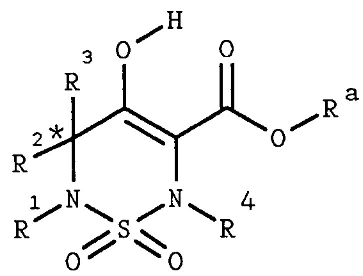

(II)

worin $\mathbf{R}^1$, $\mathbf{R}^2$, $\mathbf{R}^3$ und $\mathbf{R}^4$ die Bedeutungen gemäß Anspruch **1** haben und $\mathbf{R}^a$ einen Methyl- oder Ethylrest bedeutet, umsetzt mit einem Amin der allgemeinen Formel **III**,

$R^5R^6NH$ (III)

worin $\mathbf{R}^5$ und $\mathbf{R}^6$ die Bedeutungen gemäß Anspruch **1** haben.

**14.** Arzneimittel für die Verwendung bei Mensch und Tier, bestehend aus, oder enthaltend eine oder mehrere Verbindungen gemäß Anspruch **1**.

Europäisches Patentamt

# EUROPÄISCHER RECHERCHENBERICHT

Nummer der Anmeldung

EP 92 12 1171
PAGE1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | EP-A-0 001 113 (F.HOFFMANN-LA ROCHE & CO.)<br>* Seite 1 - Seite 3, Zeile 6 *<br>* Seite 13 *<br>--- | 1,14 | C07D513/04<br>A61K31/54 |
| A,D | US-A-3 591 584 (JOSEPH G. LOMBARDINO)<br>* Spalte 1 - Spalte 5, Zeile 73 *<br>--- | 1,14 | |
| A,D | US-A-3 223 703 (JOHN B. WRIGHT)<br>* Spalte 1 - Spalte 2, Zeile 50 *<br>--- | 1,14 | |
| A,D | JOURNAL OF MEDICINAL CHEMISTRY<br>Bd. 14, Nr. 12, Dezember 1971, COLUMBUS OHIO<br>Seiten 1171 - 1175<br>JOSEPH G.LOMBARDINO ET AL. 'Synthesis and Antiinflammatory Activity of Some 3-Carboxamides of 2-Alkyl-4-hydroxy-2H-1,2-benzothiazine 1,1-Dioxide'<br>* das ganze Dokument *<br>--- | 1,14 | |
| | | | RECHERCHIERTE SACHGEBIETE (Int. Cl.5 ) |
| A,D | JOURNAL OF MEDICINAL CHEMISTRY<br>Bd. 16, Nr. 5, Mai 1973, COLUMBUS OHIO<br>Seiten 493 - 496<br>JOSEPH G. LOMBARDINO ET AL. 'Potent Antiinflammatory N-Heterocyclic 3-Carboxamides of 4-Hydroxy-2-methyl-2H-1,2-benzothiazine 1,1-Dioxide'<br>* das ganze Dokument *<br>---<br>-/-- | 1,14 | C07D |

Der vorliegende Recherchenbericht wurde für alle Patentansprüche erstellt

| Recherchenort | Abschlußdatum der Recherche | Prüfer |
|---|---|---|
| BERLIN | 26 MAERZ 1993 | KYRIAKAKOU G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 92 12 1171
PAGE2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | CHEMICAL ABSTRACTS, vol. 109, no. 15, 10. Oktober 1988, Columbus, Ohio, US; abstract no. 129066z, Seite 701 ;Spalte 1 ; * Zusammenfassung * & ES-A-553 863 (CONSEJO SUPERIOR DE INVESTIGACIONES CIENTIFICAS) 16. November 1987<br>----- | 1,14 | |

RECHERCHIERTE SACHGEBIETE (Int. Cl.5)

Der vorliegende Recherchenbericht wurde für alle Patentansprüche erstellt

| Recherchenort | Abschlußdatum der Recherche | Prüfer |
|---|---|---|
| BERLIN | 26 MAERZ 1993 | KYRIAKAKOU G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)